(19) Europäisches Patentamt / European Patent Office / Office européen des brevets

(11) **EP 3 951 327 A1**

(12) **EUROPEAN PATENT APPLICATION**
published in accordance with Art. 153(4) EPC

(43) Date of publication:
**09.02.2022 Bulletin 2022/06**

(21) Application number: **20782573.8**

(22) Date of filing: **26.03.2020**

(51) International Patent Classification (IPC):
**G01F 1/66** (2022.01)          **A61B 5/0285** (2006.01)
**G01P 5/26** (2006.01)

(52) Cooperative Patent Classification (CPC):
**A61B 5/0285; G01F 1/66; G01P 5/26**

(86) International application number:
**PCT/JP2020/013640**

(87) International publication number:
**WO 2020/203637 (08.10.2020 Gazette 2020/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.03.2019   JP 2019068194**
**30.09.2019   JP 2019178839**

(71) Applicant: **Kyocera Corporation**
**Kyoto-shi, Kyoto 612-8501 (JP)**

(72) Inventors:
• **MATSUNAGA, Shougo**
  **Kyoto-shi, Kyoto 612-8501 (JP)**
• **TODA, Keisuke**
  **Kyoto-shi, Kyoto 612-8501 (JP)**
• **ITOU, Hiroshige**
  **Kyoto-shi, Kyoto 612-8501 (JP)**
• **HORINOUCHI, Naoki**
  **Kyoto-shi, Kyoto 612-8501 (JP)**

(74) Representative: **Viering, Jentschura & Partner mbB**
**Patent- und Rechtsanwälte**
**Am Brauhaus 8**
**01099 Dresden (DE)**

(54) **MEASURING DEVICE, MEASURING SYSTEM, MEASURING METHOD AND PROGRAM**

(57)     A measurement device includes a light emitter, a light receiver, an amplifier, and a computation processor. The light emitter irradiates, with light, an irradiation target having an internal space through which a fluid flows. The light receiver receives coherent light including light scattered by the irradiation target and outputs a signal corresponding to intensity of the coherent light. The amplifier amplifies the signal output from the light receiver. The computation processor calculates a first frequency spectrum of signal strength for each frequency with respect to a temporal strength change in the signal amplified by the amplifier, and calculates a calculation value on a flow state of the fluid with a computation including division using a first value and a second value. The first value is a value of a frequency based on the first frequency spectrum. The second value is a value of strength based on the first frequency spectrum.

FIG. 7A

FIG. 7B

```
              ┌─────────────┐
              │    Start    │
              └──────┬──────┘
                     │
                     ▼
   ┌──────────────────────────────────────┐
   │     Calculate frequency spectrum     │ ─── SP31
   └──────────────────┬───────────────────┘
                      │
                      ▼
   ┌──────────────────────────────────────┐
   │    Obtain first value and second value │ ─── SP32
   └──────────────────┬───────────────────┘
                      │
                      ▼
   ┌──────────────────────────────────────┐
   │    Calculate flow calculation value   │ ─── SP33
   └──────────────────┬───────────────────┘
                      │
                      ▼
              ┌─────────────┐
              │     End     │
              └─────────────┘
```

## Description

## FIELD

The present disclosure relates to a measurement device, a measurement system, a measurement method, and a program.

## BACKGROUND

Known devices for quantitatively measuring the flowing states of fluids include measurement devices that measure the flow rate and the flow velocity of a fluid with an optical method using, for example, a laser blood flowmeter (refer to, for example, Masaki Goma and six others, The Development of Small Laser Doppler Blood Flow Sensor, PIONEER R&D, Vol. 21, No. 1, 2012, pp 30-37 and Japanese Unexamined Patent Application Publication No. 2016-27337).

## BRIEF SUMMARY

A measurement device, a measurement system, a measurement method, and a program are described.
A measurement device according to one aspect includes a light emitter, a light receiver, an amplifier, and a computation processor. The light emitter irradiates, with light, an irradiation target having an internal space through which a fluid flows. The light receiver receives coherent light including light scattered by the irradiation target and outputs a signal corresponding to intensity of the coherent light. The amplifier amplifies the signal output from the light receiver. The computation processor calculates a first frequency spectrum of signal strength for each frequency with respect to a temporal strength change in the signal amplified by the amplifier, and calculates a calculation value on a flow state of the fluid with a computation including division using a first value and a second value. The first value is a value of a frequency based on the first frequency spectrum. The second value is a value of strength based on the first frequency spectrum.
A measurement device according to one aspect includes light emitter, a light receiver, and a computation processor. The light emitter irradiates, with light, an irradiation target having an internal space through which a fluid flows. The light receiver receives coherent light including light scattered by the irradiation target, and outputs a signal corresponding to intensity of the coherent light. The computation processor calculates a first distribution of signal strength for each frequency with respect to a temporal strength change in the signal output from the light receiver. The computation processor calculates a calculation value on a flow state of the fluid with a computation including dividing a second A-value of second signal strength in a second A-frequency range by a first A-value of first signal strength in a first A-frequency range. The first A-frequency range includes a first A-frequency having signal strength indicating a maximum value in the first distribution. The second A-frequency range is included in a frequency range higher than the first A-frequency range and includes a second A-frequency having signal strength indicating a minimum value in the first distribution.
A measurement system according to one aspect includes a light emitter, a light receiver, an amplifier, and a computation processor. The light emitter irradiates, with light, an irradiation target having an internal space through which a fluid flows. The light receiver receives coherent light including light scattered by the irradiation target, and outputs a signal corresponding to intensity of the coherent light. The amplifier amplifies the signal output from the light receiver. The computation processor calculates a first frequency spectrum of signal strength for each frequency with respect to a temporal strength change in the signal amplified by the amplifier, and calculates a calculation value on a flow state of the fluid with a computation including division using a first value and a second value. The first value is a value of a frequency based on the first frequency spectrum. The second value is a value of strength based on the first frequency spectrum.
A measurement system according to one aspect includes a light emitter, a light receiver, and a computation processor. The light emitter irradiates, with light, an irradiation target having an internal space through which a fluid flows. The light receiver receives coherent light including light scattered by the irradiation target, and outputs a signal corresponding to intensity of the coherent light. The computation processor calculates a first distribution of signal strength for each frequency with respect to a temporal strength change in the signal output from the light receiver. The computation processor calculates a calculation value on a flow state of the fluid with a computation including dividing a second A-value of second signal strength in a second A-frequency range by a first A-value of first signal strength in a first A-frequency range. The first A-frequency range includes a first A-frequency having signal strength indicating a maximum value in the first distribution. The second A-frequency range is included in a frequency range higher than the first A-frequency range and includes a second A-frequency having signal strength indicating a minimum value in the first distribution.
A measurement method according to one aspect includes performing a first process, performing a second process, and performing a third process. The first process includes irradiating, with a light emitter, an irradiation target having an internal space through which a fluid flows with light, and receiving, with a light receiver, coherent light including light scattered by the irradiation target and outputting a signal corresponding to intensity of the coherent light. The second process includes amplifying, with an amplifier, the signal output from the light receiver in the first process. The third process includes calculating, with a computation processor, a first frequency spectrum of signal strength for each frequency with respect to a temporal strength change in the signal amplified by the amplifier in the second process, and calculating a calculation value on a flow state of the fluid with a computation including division using a first value and a second value. The first value is a value of a frequency based on the first frequency spectrum. The second value is a value of strength based on the first frequency spectrum.

A measurement method according to one aspect includes performing a first A-process and performing a second A-process. The first A-process includes irradiating, with a light emitter, an irradiation target having an internal space through which a fluid flows with light, and receiving, with a light receiver, coherent light including light scattered by the irradiation target and outputting a signal corresponding to intensity of the coherent light. The second A-process includes calculating, with a computation processor, a first distribution of signal strength for each frequency with respect to a temporal strength change in the signal output from the light receiver in the first A-process. The second A-process includes calculating, with the computation processor, a calculation value on a flow state of the fluid with a computation including dividing a second A-value of second signal strength in a second A-frequency range by a first A-value of first signal strength in a first A-frequency range. The first A-frequency range includes a first A-frequency having signal strength indicating a maximum value in the first distribution. The second A-frequency range is included in a frequency range higher than the first A-frequency range and includes a second A-frequency having signal strength indicating a minimum value in the first distribution.

A program according to one aspect is a program executable by a processor included in a measurement device to cause the measurement device to function as the measurement device according to any one of the above aspects.

**BRIEF DESCRIPTION OF DRAWINGS**

FIG. 1 is a block diagram of a measurement device according to a first embodiment and a second embodiment, showing its example schematic configuration.
FIG. 2 is an example schematic partial cross-sectional view of the measurement device according to the first embodiment and the second embodiment.
FIG. 3 is a graph showing a curve Ln1 as an example of a frequency spectrum of coherent light from an irradiation target through which a fluid having a flow quantitative value of a relatively small value Q1 flows, a curve Ln2 as an example of a frequency spectrum of coherent light from an irradiation target through which a fluid having a flow quantitative value of a middle value Q2 flows, and a curve Ln3 as an example of a frequency spectrum of coherent light from an irradiation target through which a fluid having a flow quantitative value of a relatively large value Q3 flows.
FIG. 4 is a graph showing an example relationship between the flow quantitative value and a flow calculation value.
FIG. 5A is a graph showing a first example of a first value, and FIG. 5B is a graph showing a second example of the first value.
FIG. 6A is a graph showing a first example of a second value, and FIG. 6B is a graph showing a second example of the second value.
FIGs. 7A and 7B are flowcharts showing an example operation of the measurement device according to the first embodiment.
FIG. 8A is a graph showing an example frequency spectrum of coherent light calculated by the measurement device according to the second embodiment for a small flow of a fluid, and FIG. 8B is a graph showing an example frequency spectrum of coherent light calculated by the measurement device according to the first embodiment for a large flow of a fluid.
FIG. 9 is a graph showing an example relationship between a flow quantitative value and a flow calculation value.
FIGs. 10A and 10B are flowcharts showing example operations of the measurement device according to the second embodiment.
FIG. 11A is a graph of an example frequency spectrum of coherent light obtained by the measurement device for the fluid that is not flowing, FIG. 11B is a graph of an example frequency spectrum of coherent light obtained by the measurement device for the fluid that is flowing, and FIG. 11C is a graph of an example frequency spectrum obtained after noise components are reduced.
FIG. 12 is a block diagram of a measurement device according to a third embodiment, showing its example schematic configuration.
FIG. 13 is a block diagram of a measurement device according to a fourth embodiment, showing its example schematic configuration.
FIG. 14 is a block diagram of a measurement system according to a fifth embodiment, showing its example schematic configuration.
FIG. 15A is a graph showing desirable examples of a weighted frequency spectrum and a mean frequency of coherent light from an irradiation target through which fluids with respective flow rate set values of Q1, Q2, and Q3 obtained by a measurement device according to a first reference example flow, and FIG. 15B is a graph showing an example of a desirable relationship between a flow rate set value obtained by the measurement device according to the first reference example and the calculated mean frequency.

FIG. 16 is a graph showing an example of the relationship between a frequency and an amplification factor of a light receiving signal in an amplifier circuit.

FIG. 17A is a graph showing examples of a weighted frequency spectrum and a mean frequency of coherent light from an irradiation target through which fluids with respective flow rate set values of Q1, Q2, and Q3 obtained by the measurement device according to the first reference example flow, and FIG. 17B is a graph showing an example of the relationship between a flow rate set value obtained by the measurement device according to the first reference example and the calculated mean frequency.

FIG. 18 is a graph showing frequency spectra of coherent light obtained by a measurement device according to a second reference example.

FIG. 19 is a graph showing the relationship between a set value and a calculation value on the flow rate calculated by the measurement device according to the second reference example.

DETAILED DESCRIPTION

A device for measuring at least one of a flow rate or a flow velocity of a fluid using an optical method with, for example, a laser blood flowmeter is known as an example of a measurement device that quantitatively measures the flow state of a fluid. This laser blood flowmeter can calculate the blood flow rate of a living body based on, for example, changes in the wavelength of a laser beam with which the living body is irradiated due to a Doppler shift resulting from the laser beam scattered.

More specifically, in response to a living body irradiated with a laser beam with a frequency fo, the laser beam incident on the blood flowing through blood vessels (moving objects such as blood cells serving as scatterers) scatters, and the laser beam incident on other fixed tissues (including skin tissue and tissue forming the blood vessels) scatters. Such laser beams form scattered light. The diameter of the blood cells ranges from, for example, several micrometers ($\mu$m) to about 10 $\mu$m. Compared with the frequency fo of the scattered light caused by the other fixed tissues, a frequency f of the scattered light caused by the blood cells serving as scatterers is changed by $\Delta$f to a frequency fo + $\Delta$f by a Doppler shift corresponding to the movement speed of, for example, the blood cells serving as scatterers. This modulated frequency $\Delta$f is expressed with formula (1) where the velocity of the blood flow is denoted with V, the angle of incidence of a laser beam on the fluid is denoted with $\theta$, and the wavelength of the laser beam is denoted with $\lambda$.

$$\Delta f = (2V \times \cos\theta)/\lambda \qquad (1)$$

Mutual interference between the scattered light with the frequency fo caused by the fixed tissues and the scattered light with the frequency fo + $\Delta$f caused by the moving blood cells enables observation of a difference frequency $\Delta$f in the form of an optical beat (beat). In other words, a signal (light receiving signal) obtained by receiving these two types of scattered light contains a component of a signal (also referred to as an optical beat signal) corresponding to the optical beat caused by mutual interference of these two types of scattered light.

The difference frequency $\Delta$f corresponding to the frequency of the optical beat is far lower than the frequency f of the original light. For example, the original light with a wavelength of 780 nm has a frequency of about 400 terahertz (THz), which exceeds the response speed detectable by a normal photodetector. In contrast, although depending on the movement speed of the blood cells, the frequency $\Delta$f of the optical beat (also referred to as an optical beat frequency) is, for example, within the range of about several kilohertz (kHz) to about several tens of kHz and is thus within a frequency range fully responsive and detectable by a normal photodetector. Thus, a signal (light receiving signal) obtained by the photodetector receiving the scattered light with the frequency fo scattered by the fixed tissues and the scattered light with the frequency fo + $\Delta$f scattered by the moving blood cells indicates a wave form obtained by superimposing an intensity modulated signal with the optical beat frequency $\Delta$f on a direct-current (DC) component signal (DC signal). Then, the optical beat signal with the frequency $\Delta$f is analyzed to calculate the blood flow rate.

For example, a frequency spectrum P(f) for a light receiving signal detected by the photodetector is first calculated using a computation such as a Fourier transform (FFT). Subsequently, the frequency spectrum P(f) is weighted with the frequency f to calculate a weighted frequency spectrum P(f) $\times$ f. Then, the weighted frequency spectrum P(f) $\times$ f is integrated within a predetermined frequency range to calculate a first calculation value ($\int$$\{$P(f) $\times$ f$\}$df). Subsequently, as shown in formula (2), the first calculation value ($\int$f$\cdot$P(f)df) is divided by a second calculation value ($\int$P(f)df) calculated by integrating the frequency spectrum P(f) within the predetermined frequency range to calculate a mean frequency fm, which is multiplied by a predetermined constant to calculate the blood flow rate of a living body.

$$fm = \int\{P(f) \times f\}df/\{\int P(f)df\} \qquad (2)$$

In this example, a fluid in which light scatterers of about several micrometers are dispersed flows through a transparent tube serving as a flow passage, and a flow rate Q of the fluid is measured with a laser blood flowmeter. In this structure, the flow rate (also referred to as a flow rate set value) of the fluid flowing through the flow passage can be set with, for example, a pump. For example, the flow rate set value is increased to Q1, Q2, and Q3 in this order, and the frequency spectrum P(f) for the optical beat signal, the weighted frequency spectrum P(f) $\times$ f, the mean frequency fm, and the flow rate Q of the fluid are calculated with the laser blood flowmeter for each of the flow rate set values Q1, Q2, and Q3.

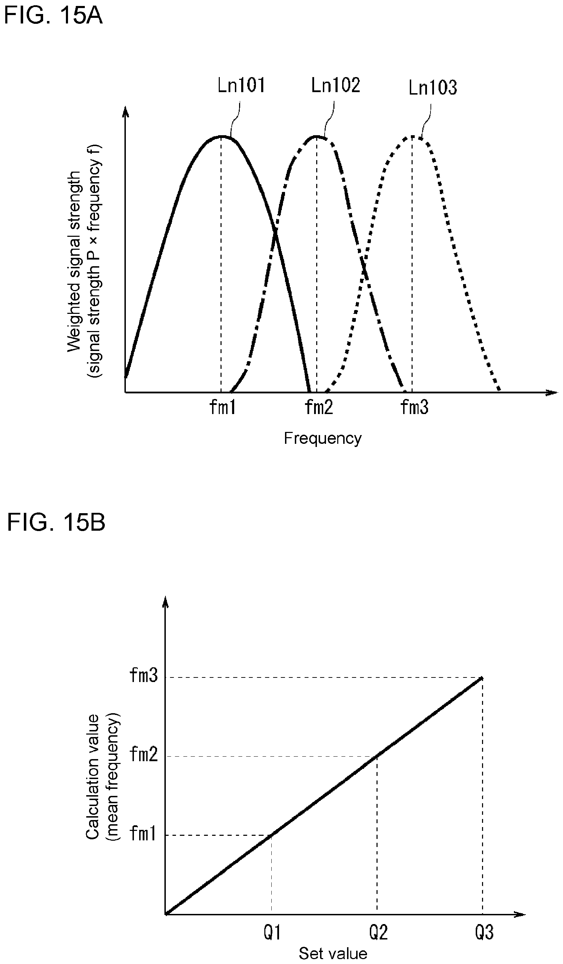

For the flow rate set value Q1, for example, a mean frequency fm1 is calculated based on the weighted frequency spectrum P(f) × f indicated with a curve Ln101 indicated with a bold solid line in FIG. 15A. For the flow rate set value Q2, a mean frequency fm2 is calculated based on the weighted frequency spectrum P(f) × f indicated with a curve Ln102 indicated with a bold dot-dash line in FIG. 15A. For the flow rate set value Q3, a mean frequency fm3 is calculated based on the weighted frequency spectrum P(f) × f indicated with a curve Ln103 indicated with a bold broken line in FIG. 15A. For example, as indicated with a bold solid line in FIG. 15B, if the flow rate set values Q1, Q2, and Q3 and the mean frequencies fm1, fm2, and fm3 are proportional to each other, the flow rate Q of the fluid can be correctly calculated by multiplying the mean frequency fm by a predetermined multiplier.

The photodetector outputs a weak light receiving signal. Thus, the laser blood flowmeter is assumed to amplify the light receiving signal with, for example, an amplifier circuit, and then calculate the frequency spectrum P(f) for the optical beat signal, the weighted frequency spectrum P(f) × f, the mean frequency fm, and the flow rate Q of the fluid.

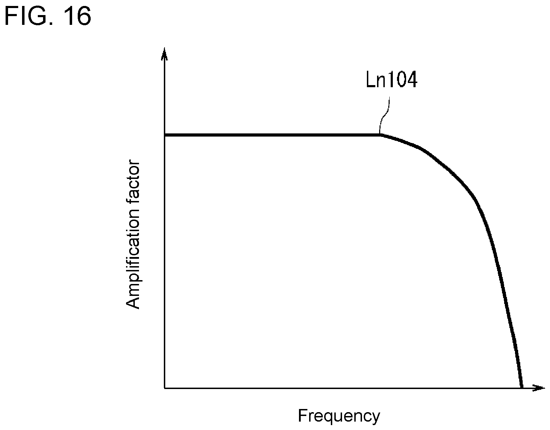

However, for example, as indicated with a curve Ln104 indicated with a bold solid line in FIG. 16, the amplification factor of the light receiving signal in the amplifier circuit depends on the frequency of the light receiving signal. More specifically, the amplifier circuit has a lower amplification factor of the signal strength as the frequency of the light receiving signal increases. Thus, although an increase in the flow rate set value shifts the frequency components forming the light receiving signal toward higher frequencies, higher frequency components in the weighted frequency spectrum P(f) × f attenuate. Thus, for example, the mean frequency of the frequency spectrum P(f) calculated using the weighted frequency spectrum P(f) × f may fail to increase in proportion to the increase in the flow rate set value.

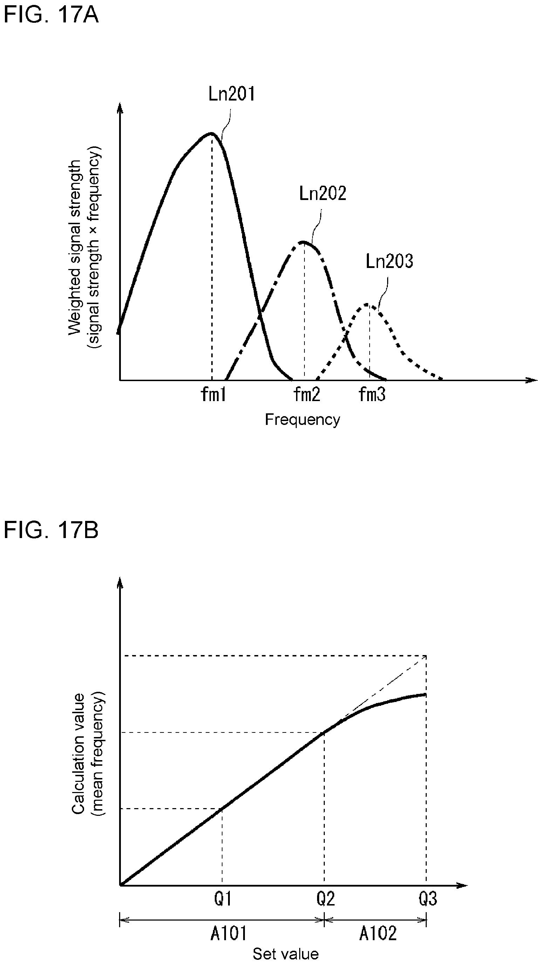

In this example, the flow rate set value is increased to Q1, Q2, and Q3 in this order. For the flow rate set value Q1, a mean frequency fm1 is calculated based on the weighted frequency spectrum P(f) × f indicated with a curve Ln201 indicated with a bold solid line in FIG. 17A. For the flow rate set value Q2, a mean frequency fm2 is calculated based on the weighted frequency spectrum P(f) × f indicated with a curve Ln202 indicated with a bold dot-dash chain line in FIG. 17A. For the flow rate set value Q3, a mean frequency fm3 is calculated based on the weighted frequency spectrum P(f) × f indicated with a curve Ln203 indicated with a bold broken line in FIG. 17A.

As indicated with a bold solid line in FIG. 17B, the flow rate set value within a relatively small range A101 and the mean frequency are proportional to each other, but the flow rate set value within a relatively large range A102 and the mean frequency are not proportional to each other. FIG. 17B shows the relationship between the flow rate set value and the mean frequency in a desirable proportional relation with a thin two-dot chain line for comparison. More specifically, with respect to the increase rate of the flow rate set value within the relatively large range A102, the increase rate of the calculated mean frequency fm decreases. More specifically, for example, despite an increase in the flow rate set value, the mean frequency obtained by the laser blood flowmeter may fail to increase in proportion to the increase in the flow rate set value. Thus, the flow rate Q of a fluid may fail to be correctly calculated by multiplying the mean frequency fm by a predetermined multiplier.

For example, the blood flow rate of a living body is calculated by calculating the frequency spectrum P(f) with a computation such as an FFT on a signal component corresponding to beat signal light contained in a light receiving signal detected by the photodetector. The blood flow rate can be calculated by multiplying, for example, the value (also referred to as a flow rate calculation value) Q4 calculated with formula (3) by predetermined coefficients L and K. For example, the frequency f within a range of 0 to 500 kHz is integrated with formula (3).

$$Q4 = \int \{f \times P(f)\} df \qquad (3)$$

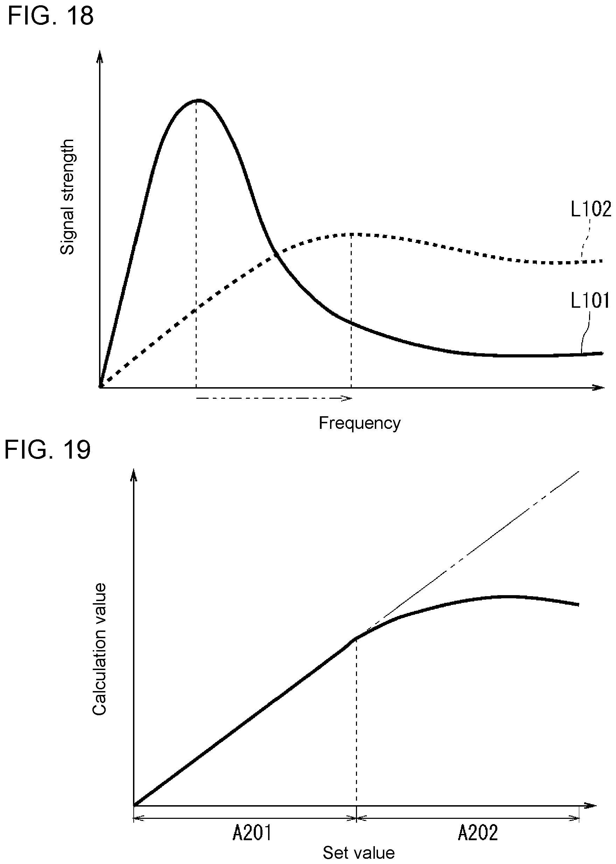

In this example, the flow rate of a fluid in which light scatterers of about several micrometers are dispersed is measured with a laser blood flowmeter if the fluid flows through a transparent tube serving as a flow passage. For example, the frequency spectrum of a beat signal obtained using the laser blood flowmeter is calculated. In this example, the flow rate (also referred to as a flow rate set value) of a fluid flowing through a flow passage can be set with, for example, a pump. If, for example, the flow rate set value is a relatively small value Qa, a frequency spectrum indicated with a bold solid line L101 in FIG. 18 is obtained. If the flow rate set value is, for example, a relatively large value Qb, a frequency spectrum indicated with a bold broken line L102 in FIG. 18 is obtained. In the frequency spectrum, in response to, for example, an increase in the flow rate set value, the signal strength of a low-frequency component decreases and the signal strength of a high-frequency component increases. However, the signal strength of the high-frequency component in the frequency spectrum does not greatly increase due to, for example, the effect of environmental noise or the rate controlling of a sampling frequency of the light receiving signal in a microcomputer.

Thus, for example, despite an increase in the flow rate set value, the flow rate calculation value calculated based on the frequency spectrum of the light receiving signal obtained by the laser blood flowmeter and the computation with formula (3) may fail to increase in proportion to the increase in the flow rate set value. For example, as shown in FIG. 19, the flow rate set value within a relatively low range A201 and the flow rate calculation value have a linear relationship. However, the flow rate set value within a relatively high range A202 and the flow rate calculation value may fail to have a linear relationship. For example, the flow rate calculation value increases in proportion to the increase in a relatively low flow rate set value, but the increase in the flow rate calculation value with respect to the increase in a relatively high flow rate set value gradually decreases, and the flow rate calculation value fails to increase in proportion to the increase in the flow rate set value.

The issues described above occur not only to the measurement device that measures the flow rate of a fluid, but also are common in general measurement devices that measure quantitative values on the flow state of a fluid including at least one of a flow rate or a flow velocity of the fluid.

Thus, measurement devices that quantitatively measure the flow state of a fluid are to improve the measurement accuracy.

The inventors of the present invention have created a technology of improving measurement accuracy of a measurement device that quantitatively measures the flow state of a fluid.

First to fifth embodiments will now be described below with reference to the drawings. Throughout the drawings, the components having the same structures and functions are given the same reference numerals, and will not be described repeatedly. The drawings are schematic.

1. First Embodiment

1-1. Measurement Device

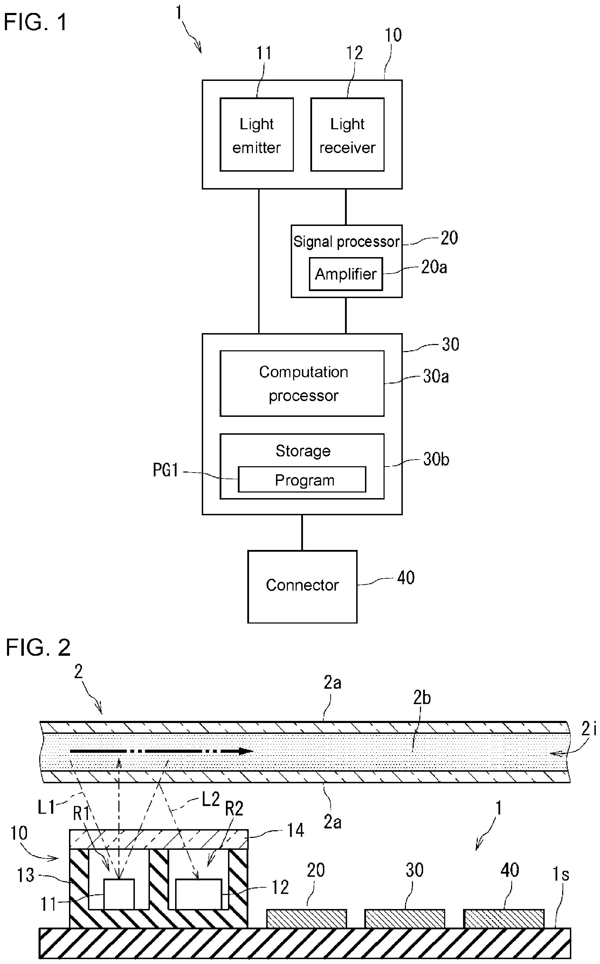

As shown in FIGs. 1 and 2, a measurement device 1 according to a first embodiment can quantitatively measure, for example, the flow state of a fluid 2b that flows through an internal space 2i of an object (also referred to as a flow passage component) 2a forming a flow passage. The flow passage component 2a may include, for example, a tubular object (also referred to as a tubular body) such as a blood vessel in a living body or pipes in various devices. The quantitative values (also referred to as flow quantitative values) indicating the flow state of the fluid 2b may include, for example, at least one of the flow rate or the flow velocity. The flow rate is the quantity of a fluid at which the fluid passes through a flow passage per unit time. The quantity of the fluid may be expressed in, for example, a volume or a mass. The flow velocity is the velocity of the fluid flowing through the flow passage. The flow velocity may be expressed with a distance by which the fluid flows per unit time.

The measurement device 1 according to the first embodiment can quantitatively measure the flow state of the fluid 2b with, for example, the Doppler effect for light. For example, if light with which the fluid 2b is irradiated is scattered by the fluid 2b, the Doppler effect corresponding to the flow of the fluid 2b causes a shift (also referred to as a Doppler shift) of the frequency of light corresponding to the movement speed of the fluid 2b. The measurement device 1 according to the first embodiment can measure the flow quantitative value indicating the flow state of the fluid 2b with this Doppler shift. The components of the measurement device 1 described later can be manufactured with any known methods as appropriate.

Examples of the fluid 2b serving as a target (also referred to as a measurement target) having its flow state quantitatively measured include the fluid 2b that scatters light, and a fluid that allows a substance that scatters light (also referred to as a scatter substance) or an object that scatters light (also referred to as a scatterer) to flow through the fluid. More specifically, examples of the fluid 2b serving as a measurement target include water, blood, printer ink, or gas containing a scatterer such as powder. If a scatter substance or a scatterer flows with the fluid, the flow rate of the scatter substance or the scatterer may be used as the flow rate of a fluid, or the flow velocity of the scatter substance or the scatterer may be used as the flow velocity of the fluid.

As shown in FIGs. 1 and 2, the measurement device 1 according to the first embodiment includes, for example, a sensor 10, a signal processor 20, and a controller 30. The measurement device 1 also includes a connector 40.

The sensor 10 includes, for example, a light emitter 11 and a light receiver 12.

The light emitter 11 can irradiate, with light L1, an object (also referred to as an irradiation target) 2 having the internal space 2i through which the fluid 2b flows. The irradiation target 2 includes at least an object (flow passage component) 2a forming a flow passage of a tubular body, and a fluid 2b flowing through the flow passage. Examples of the light L1 with which the irradiation target 2 is irradiated by the light emitter 11 include light having a predetermined wavelength suitable for the fluid 2b serving as a measurement target. For example, if the fluid 2b is blood, the irradiation target 2 is irradiated with the light L1 having a wavelength set to about 600 to 900 nanometers (nm). For example, if the fluid 2b is printer ink, the irradiation target 2 is irradiated with the light having a wavelength set to about 700 to 1000 nm. A semiconductor laser device such as a vertical-cavity surface-emitting laser (VCSEL) is used as an example of the light emitter 11.

The light receiver 12 can receive coherent light L2 including light scattered by the irradiation target 2 in the light L1 with which the irradiation target 2 is irradiated by the light emitter 11. For example, the light receiver 12 can convert the received light to an electric signal corresponding to the light intensity. In other words, the light receiver 12 can receive the coherent light L2 including light scattered by the irradiation target 2, and output a signal corresponding to the intensity of the coherent light L2. The coherent light L2 that can be received by the light receiver 12 includes coherent light, in the light scattered by the irradiation target 2, caused by scattered light without a Doppler shift from an object stationary around the fluid 2b (also referred to as a stationary object) and scattered light with a Doppler shift with a shift amount of $\Delta f$ from the fluid 2b. For blood flowing through a blood vessel serving as an example of the fluid 2b, the stationary object includes the skin and the blood vessels. For ink flowing through a pipe serving as an example of the fluid 2b, the stationary object includes an object (flow passage component) 2a forming a flow passage for the fluid 2b such as a pipe. In this case, the pipe may be formed from, for example, a translucent material. Examples of the translucent material include glass and polymer resin.

A change in the intensity of the coherent light L2 with time (also referred to as a temporal change) can indicate a beat of the frequency corresponding to a difference (also referred to as a difference frequency) $\Delta f$ between the frequency of scattered light without a Doppler shift and the frequency of scattered light with a Doppler shift. Thus, a signal output from the light receiver 12 and corresponding to the intensity of the coherent light L2 can contain a component of a signal corresponding to the beat (also referred to as a beat signal or an optical beat signal) with respect to the temporal change in the intensity of the coherent light L2. A device that can follow the beat (also referred to as having time resolution) with respect to the temporal change in the intensity of the coherent light L2 is usable as an example of the light receiver 12. The wavelength of light that can be received by the light receiver 12 can be set in accordance with the measurement conditions such as the wavelength of the light L1 with which the irradiation target 2 is irradiated by the light emitter 11 and the velocity range of the fluid 2b. Examples of the light receiver 12 include various photodiodes including a silicon (Si) photodiode, a gallium arsenide (GaAs) photodiode, an indium gallium arsenide (InGaAs) photodiode, and a germanium (Ge) photodiode.

The sensor 10 may also include a package 13. The package 13 accommodates the light emitter 11 and the light receiver 12. In the example in FIG. 2, the measurement device 1 includes a substrate (also referred to as a mounting board) 1s that receives the sensor 10, the signal processor 20, the controller 30, and the connector 40. Examples of the mounting board 1s include a printed circuit board. The package 13 in the sensor 10 is located on the mounting board 1s. The mounting board 1s electrically connects, for example, the sensor 10 to the signal processor 20, the signal processor 20 to the controller 30, the sensor 10 to the controller 30, and the controller 30 to the connector 40.

The package 13 has, for example, a cubic or rectangular parallelepiped external shape. The package 13 includes, for example, a first recess R1 and a second recess R2 open upward. The first recess R1 receives the light emitter 11. The second recess R2 receives the light receiver 12. The light L1 emitted from the light emitter 11 is applied to the irradiation target 2 through the opening of the first recess R1. The coherent light L2 from the irradiation target 2 is received by the light receiver 12 through the opening of the second recess R2. The package 13 may be, for example, a multilayered wiring board formed from ceramic or organic materials. Examples of the ceramic material include sintered aluminum oxide and sintered mullite. Examples of the organic material include an epoxy resin and a polyimide resin.

As shown in FIG. 2, a translucent cover 14 may be located to cover the openings of the first recess R1 and the second recess R2 in the package 13. This structure can hermetically seal the light emitter 11 in the first recess R1 in the package 13, and the light receiver 12 in the second recess R2 in the package 13. The cover 14 may be a glass plate.

The signal processor 20 can perform various processes on an electric signal received from the light receiver 12. Examples of the various processes may include conversion of an electric signal into a voltage, amplification of the strength of an electric signal, and conversion of an analog signal to a digital signal. Thus, the signal processor 20 functions as, for example, a unit 20a that can amplify a signal (also referred to as an amplifier) output from the light receiver 12. The signal processor 20 including, for example, an amplifier circuit can implement a function of the amplifier 20a. The amplification factor of the signal in the amplifier 20a may be set to be within a range of, for example, 3 to 150 decibels (dB). The frequency at which the amplification factor of a signal starts attenuating (also referred to as a cut-off frequency) in the amplifier 20a may be set to, for example, 500 kHz. The amplifier 20a may amplify a signal in, for example, two, three, or more stages. The electric signal output from the light receiver 12 contains, for example, a DC component and an alternating-current (AC) component. Thus, the signal processor 20 may divide the electric signal output from the light receiver 12 into a DC component and an AC component, and then amplify the AC component signal with the amplifier 20a. The various processes performed by the signal processor 20 may include conversion of an electric signal to a voltage, division of an electric signal into AC and DC components, amplification of the AC signal, and conversion of an analog signal to a digital signal. The signal processor 20 includes multiple electronic components including active elements such as a transistor or a diode and a passive element such as a capacitor. The signal processor 20 may include a circuit such as a current-voltage conversion circuit (I-V conversion circuit), an AC-DC separation circuit (AC-DC decoupling circuit), an AC amplifier circuit, and an analog-to-digital conversion circuit (AD conversion circuit). The signal processor 20 can perform processing such as amplification and AD conversion on an analog electric signal received from the light receiver 12, and then output a digital signal to the controller 30.

The controller 30 can control, for example, the measurement device 1. The controller 30 includes, for example, multiple electronic components including an active element such as a transistor or a diode and a passive element such as a capacitor. The connector 40 can electrically connect, for example, the controller 30 to external devices.

For example, multiple electronic components may be integrated to form one or more integrated circuits (ICs) or large-scale integration circuits (LSIs) to form various functional units including the signal processor 20, the controller 30, and the connector 40. Multiple ICs or LSIs may be further integrated to form various functional units including the signal processor 20, the controller 30, and the connector 40. Multiple electronic components forming the signal processor 20, the controller 30, and the connector 40 are mounted on, for example, the mounting board 1s. The package 13 and each of the signal processor 20, the controller 30, and the connector 40 are electrically connected.

The controller 30 includes, for example, a computation processor 30a and a storage 30b.

The computation processor 30a includes, for example, a processor serving as an electric circuit. The processor may include, for example, one or more processors, a controller, a microprocessor, a microcontroller, an application-specific integrated circuit (ASIC), a digital signal processor, a programmable logic device, a combination of any of these devices or components, or a combination of any other known devices or components.

The storage 30b includes, for example, a random-access memory (RAM) and a read-only memory (ROM). The storage 30b stores, for example, firmware containing a program PG1. The computation processor 30a can perform computation or processing on one or more pieces of data in accordance with the firmware stored in the storage 30b. In other words, for example, the computation processor 30a executing the program PG1 enables implementation of the various functions of the measurement device 1. Thus, the controller 30 can control, for example, the operation of the light emitter 11.

The frequency and the strength (also referred to as signal strength) of an electric signal output from the light receiver 12 depend on the Doppler effect for light. Thus, the frequency spectrum showing the relationship between the frequency and the strength of the electric signal changes in accordance with the flow quantitative value (flow rate or flow velocity) of the fluid 2b. Thus, the controller 30 can perform computation to quantitatively measure the flow state of the fluid 2b based on the electric signal output from the light receiver 12 and then amplified by the signal processor 20 with the computation processor 30a.

The computation processor 30a can calculate the distribution of the signal strength for each frequency with respect to changes in the signal over time (temporal strength change). The signal is obtained by amplifying the signal output from the light receiver 12 with the amplifier 20a. The computation processor 30a performs an analysis with a computation such as a Fourier transform on a temporal strength change in a signal output from the signal processor 20. Thus, a power spectrum (also referred to as a first frequency spectrum) indicating the distribution of the signal strength for each frequency with respect to the temporal strength change in the signal amplified by the amplifier 20a can be calculated. The frequency range in the first frequency spectrum can be set based on, for example, a sampling rate in an AD conversion circuit.

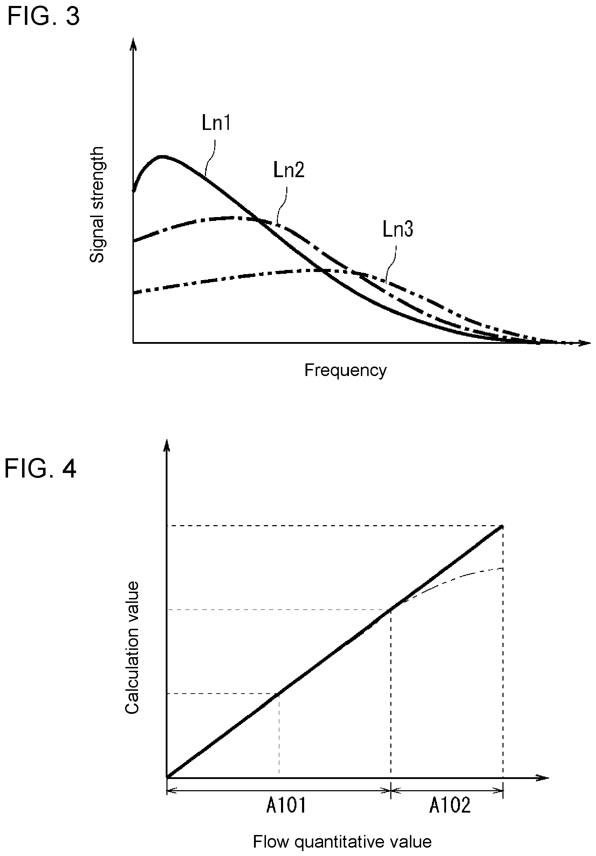

For example, for the flow quantitative value (flow rate or flow velocity) of the fluid 2b with a relatively small value Q1, the computation processor 30a can calculate a first frequency spectrum indicated with a curve Ln1 indicated with a bold solid line in FIG. 3. For the flow quantitative value (flow rate or flow velocity) of the fluid 2b with a relatively intermediate value Q2, the computation processor 30a can calculate a first frequency spectrum indicated with a curve Ln2 indicated with a bold dot-dash chain line in FIG. 3. For example, for the flow quantitative value (a flow rate or a flow velocity) of the fluid 2b with a relatively large value Q3, the computation processor 30a can calculate a first frequency spectrum indicated with a curve Ln3 indicated with a bold two-dot chain line in FIG. 3. In response to an increase in the flow quantitative value of the fluid 2b, the signal strength gradually increases or decreases with a change in the frequency to form the shape of the first frequency spectrum P(f) as shown in FIG. 3.

Calculating Flow Calculation Value

For example, based on the first frequency spectrum, the computation processor 30a can determine a first value V1 on the frequency and a second value V2 on the signal strength. The computation processor 30a can calculate a calculation value (also referred to as a flow calculation value) Vc on the flow state of the fluid 2b flowing through the internal space 2i of the irradiation target 2 with a computation including division using the first and second values V1 and V2. For example, the flow calculation value Vc can be calculated with formula (4), the first value V1, and the second value V2. The division using the first and second values V1 and V2 includes dividing the first value V1 by the second value V2. The flow state may include at least one of the flow rate or the flow velocity.

$$Vc = V1/V2 \qquad (4)$$

In this example, the measurement device 1 calculates the flow calculation value Vc while the quantitative value (flow quantitative value) on the flow state of the fluid 2b flowing through a transparent tube serving as the flow passage component 2a is set with, for example, a pump. As shown in FIG. 4, the flow calculation value Vc can indicate an increase in proportion to an increase in the flow quantitative value.

As shown in FIG. 16, in the amplifier circuit, the amplification factor of a signal decreases with an increase in the frequency of the signal. Thus, as shown in, for example, FIGs. 17A and 17B, the mean frequency of the first frequency spectrum tends to fail to increase in proportion to an increase in the flow quantitative value. As shown in FIG. 3, an increase in the flow quantitative value shifts the frequency component forming the first frequency spectrum toward a higher frequency but attenuates a higher frequency component in the first frequency spectrum. Thus, despite an increase in the flow quantitative value, the increase rate of the first value V1 on the frequency based on the first frequency spectrum tends to fall below the increase rate of the flow quantitative value. In addition, the second value V2 on the strength based on the first frequency spectrum tends to decrease in accordance with an increase in the flow quantitative value, as in the increase rate of the first value V1. Calculating the flow calculation value Vc using formula (4) with these tendencies yields the flow calculation value Vc for which the decrease in the increase rate of the first value V1 with respect to the increase rate of the flow quantitative value cancels the decrease in the second value V2 with respect to the increase in the flow quantitative value. In other words, using the first and second values V1 and V2 enables a computation in which the values attenuating in accordance with amplification characteristics of the amplifier 30a cancel each other. As indicated with a bold line in FIG. 4, the flow calculation value Vc can indicate an increase in proportion to an increase in the flow quantitative value. In other words, the flow quantitative value from the relatively low range to the relatively high range and the flow calculation value Vc have a linear relationship. Thus, the measurement device 1 that quantitatively measures the flow state of a fluid can improve the measurement accuracy. FIG. 4 shows the relationship between the flow rate set value and the flow calculation value (mean frequency) shown in FIG. 17B with a thin two-dot chain line for comparison.

For example, a specific value of the frequency for the first frequency spectrum can be used as the first value V1 on the frequency based on the first frequency spectrum. For example, a specific value of the frequency for the frequency spectrum (also referred to as a second frequency spectrum) calculated through various computations on the first frequency spectrum may be used as the first value V1 on the frequency based on the first frequency spectrum. For example, a specific value of the strength for the first frequency spectrum can be used as the second value V2 on the strength based on the first frequency spectrum. For example, a specific value of the strength for the second frequency spectrum may be used as the second value V2 on the strength based on the first frequency spectrum. The various computations may include, for example, a weighting computation using a frequency for the first frequency spectrum. In this case, the computation processor 30a can calculate, for example, a weighted spectrum (second frequency spectrum) by a weighting computation including multiplying the strength for each frequency in the first frequency spectrum by the corresponding frequency.

Specific Example of First Value V1

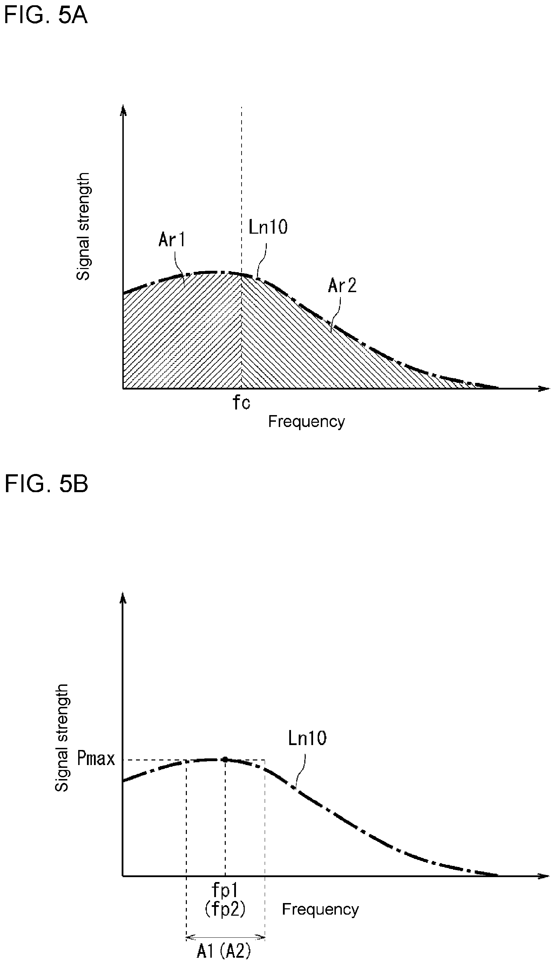

Examples of the first value V1 can include a boundary frequency in which a value (also referred to as a first integrated value) obtained by integration on the strength of lower frequencies and a value (also referred to as a second integrated value) obtained by integration on the strength of higher frequencies in either the first or second frequency spectrum have a predetermined ratio. As shown in FIG. 5A, the computation processor 30a calculates an integrated value (first integrated value) Ar1 of the signal strength of frequencies lower than a frequency fc and an integrated value (second integrated value) Ar2 of the signal strength of frequencies higher than the frequency fc in the frequency spectrum. FIG. 5A shows an example of a frequency spectrum with a curve Ln10 indicated with a bold dot-dash chain line. Subsequently, the computation processor 30a determines the frequency f at which the first integrated value Ar1 and the second integrated value Ar2 have a predetermined ratio as a boundary frequency. The computation processor 30a then uses, for example, the frequency f serving as a boundary frequency as the first value V1. The predetermined ratio between the first integrated value Ar1 and the second integrated value Ar2 is set to be between 2:3 to 3:2. For example, the predetermined ratio between the first integrated value Ar1 and the second integrated value Ar2 may be set to be 1:1.

In addition, examples of the first value V1 may include a frequency with any of strength (also referred to as first strength) in a frequency range (also referred to as a first frequency range) A1 including a frequency (also referred to as a first frequency) fp1 indicating maximum strength (also referred to as first maximum strength) Pmax in either the first or second frequency spectrum. As shown in FIG. 5B, the first frequency range A1 may be set to a range of a predetermined width range with reference to the first frequency fp1 at which the strength of the frequency spectrum indicates the maximum value Pmax. FIG. 5B shows an example of the frequency spectrum with a curve Ln10 indicated with a bold dot-dash chain line. More specifically, the first frequency range A1 can be set to, for example, a range with a predetermined width with the first frequency fp1 at the center. The first strength may be larger than or equal to 0.8 or 0.9 times the first maximum strength Pmax. As shown in FIG. 5B, the computation processor 30a determines the first frequency fp1 at which the signal strength indicates a maximum value Pmax in the frequency spectrum. The computation processor 30a then uses, for example, the frequency for the first strength in the first frequency range A1 including the first frequency fp1 as the first value V1. If the first strength is the first maximum strength Pmax, the first strength can be easily obtained. Thus, the flow quantitative value ranging from a small to a large value and the flow calculation value Vc easily have an approximate proportional relation. Thus, the measurement device 1 can easily improve the measurement accuracy. As shown in FIG. 5B, the frequency with the first strength is the first frequency fp1. As shown in FIG. 5B, the computation processor 30a determines the first frequency fp1 at which the signal strength has a maximum value Pmax in the frequency spectrum. The computation processor 30a then uses, for example, the first frequency fp1 as the first value V1.

Examples of the first value V1 may include a frequency with any inclination (also referred to as a first inclination) in a frequency range (also referred to as a second frequency range) A2 including a frequency (also referred to as a second frequency) fp2 in which the absolute value of inclination of a strength change indicates a minimum value in either the first or second frequency spectrum. The ratio of a change in the signal strength to the change in frequency is used as an example of the inclination of a strength change in the frequency spectrum. As shown in FIG. 5B, for example, the second frequency range A2 can be set to a predetermined width range with reference to the second frequency fp2 at which the absolute value of the strength inclination indicates a minimum value (for example, 0). More specifically, the second frequency range A2 can be set to a predetermined width range having the second frequency fp2 at the center. An inclination with an absolute value of a predetermined value or smaller can be used as an example of the first inclination. In this case, as shown in FIG. 5B for example, the computation processor 30a determines the second frequency fp2 at which the inclination of the signal strength has a minimum absolute value in the frequency spectrum. The computation processor 30a then uses, for example, the frequency with the first inclination in the second frequency range A2 including the second frequency fp2 as the first value V1. If the first inclination has a minimum absolute value of inclination of a strength change, the first inclination can be easily obtained. Thus, the flow quantitative value ranging from a small to a large value and the flow calculation value Vc easily have an approximate proportional relation. Thus, the measurement device 1 can easily improve the measurement accuracy. In this case, as shown in FIG. 5B for example, the frequency with the first inclination is the second frequency fp2. As shown in FIG. 5B, the computation processor 30a determines the second frequency fp2 at which the inclination of a signal strength change indicates a minimum absolute value in the frequency spectrum. The computation processor 30a thus uses, for example, the second frequency fp2 as the first value V1.

The first value V1 may be a frequency shift amount Δf between the frequency fo of the light L1 with which the irradiation target 2 is irradiated by the light emitter 11 and the frequency of the coherent light L2 including light scattered by the irradiation target 2. For example, a signal corresponding to the intensity of the coherent light L2 output from the light receiver 12 contains an optical beat signal component including the frequency corresponding to the frequency shift amount Δf. The first frequency spectrum is, for example, a power spectrum indicating the distribution of the signal strength for each frequency with respect to the temporal strength change in the signal amplified by the amplifier 20a after being output from the light receiver 12. Thus, the frequency shift amount Δf can be estimated, for example, based on the first frequency spectrum. The frequency shift amount Δf is an example of a frequency value based on the first frequency spectrum. The frequency shift amount Δf may be a mean frequency fm for the first frequency spectrum or a frequency range width (also referred to as a first frequency width) indicating the strength of a specific value or higher in the first frequency spectrum. In this case as well, the flow quantitative value ranging from a small to a large value and the flow calculation value Vc have an approximate proportional relation. Thus, the measurement device 1 can improve the measurement accuracy. The mean frequency fm corresponds to, for example, the frequency of an optical beat signal corresponding to the frequency shift amount Δf. The mean frequency fm can be calculated by, for example, dividing the integrated value ($\int\{P(f) \times f\}df$) of the strength for the second frequency spectrum $P(f) \times f$ obtained by weighting the first frequency spectrum $P(f)$ with the frequency f by the integrated value ($\int P(f)df$) of the strength for the first frequency spectrum $P(f)$. The computation processor 30a calculates, for example, the second frequency spectrum $P(f) \times f$ by weighting the first frequency spectrum $P(f)$ with the frequency f. Subsequently, the computation processor 30a calculates, for example, the integrated value ($\int\{P(f) \times f\}df$) of the strength for the second frequency spectrum $P(f) \times f$ and the integrated value ($\int P(f)df$) of the strength for the first frequency spectrum $P(f)$. Then, the computation processor 30a calculates, for example, the mean frequency fm by dividing the integrated value ($\int\{P(f) \times f\}df$) of the strength for the second frequency spectrum $P(f) \times f$ by the integrated value ($\int P(f)df$) of the strength for the first frequency spectrum $P(f)$. The computation processor 30a then uses, for example, the mean frequency fm as the first value V1. Similarly to the mean frequency fm, the first frequency width has a strong correlation with the quantitative value (flow quantitative value) indicating the flow state of the fluid 2b flowing through the internal space 2i of the flow passage component 2a. Thus, instead of the mean frequency fm, for example, the first frequency width in the first frequency spectrum can be used as the value corresponding to the frequency shift amount Δf. A value with reference to the maximum value Pmax of the strength in the first frequency spectrum is used as an example specific value for defining the first frequency width. More specifically, a half of the maximum value Pmax of the strength in the first frequency spectrum is used as an example of the specific value. In other words, a half width may be used as a first frequency width. In this case, for example, the computation processor 30a calculates the first frequency width. The computation processor 30a then uses, for example, the first frequency width as the first value V1.

The first value V1 may also be a value of the frequency calculated by one or more calculations such as multiplication by a coefficient or exponentiation on the above-described boundary frequency, the frequency with the first strength, the frequency with the first inclination, or the frequency shift amount Δf. In other words, the value of the frequency selected from the above-described boundary frequency, the frequency with the first strength, the frequency with the first inclination, and the frequency shift amount Δf may be used as an example of the first value V1. In another example, a value of at least one frequency selected from the above-described boundary frequency, the frequency with the first strength, the frequency with the first inclination, and the frequency shift amount Δf may be used as an example of the first value V1. In such structures as well, the flow quantitative value ranging from a small to a large value and the flow calculation value Vc have an approximate proportional relation. Thus, the measurement device 1 can improve the measurement accuracy. The sum of two or more values selected from the above-described boundary frequency, the frequency with the first strength, the frequency with the first inclination, and the frequency shift amount Δf may be used as an example of the first value V1.

Specific Example of Second Value V2

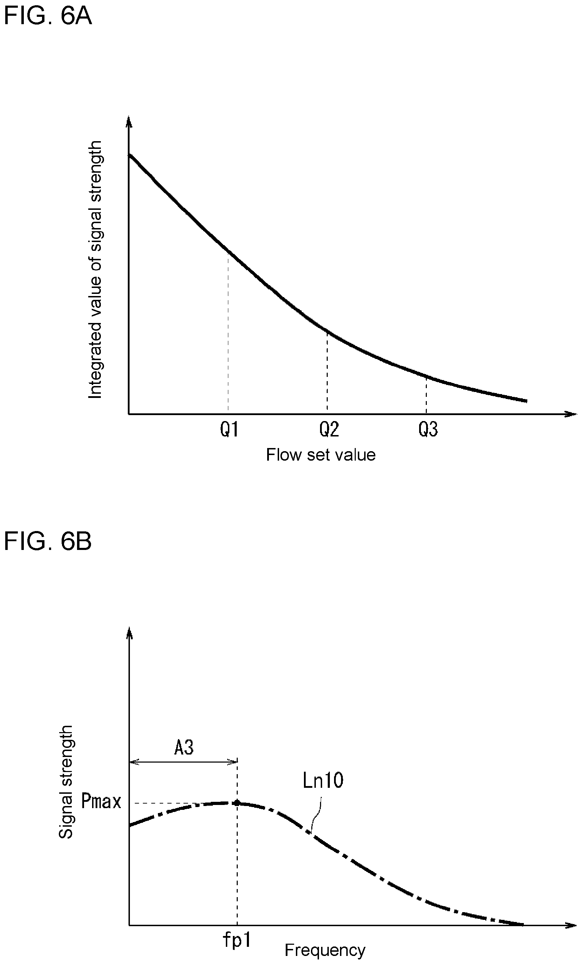

The second value V2 may be an integrated value (also referred to as a third integrated value) obtained by integration on either the first or second frequency spectrum. In this case, the computation processor 30a may calculate the third integrated value by, for example, calculating the integrated value ($\int P(f)df$) of the strength for the first frequency spectrum P(f). For example, as shown in FIG. 6A, the integrated value ($\int P(f)df$) of the strength for the first frequency spectrum P(f) tends to decrease with an increase in a flow set value. The computation processor 30a may calculate the second frequency spectrum P(f) × f by, for example, weighting the first frequency spectrum P(f) with the frequency f. The computation processor 30a may calculate the third integrated value by, for example, calculating the integrated value ($\int \{P(f) \times f\}df$) of the strength for the second frequency spectrum P(f) × f. Integration on the frequency spectrum for calculating the third integrated value may be performed on, for example, a predetermined frequency range. The predetermined frequency range may be set to be lower than or equal to a preset specific frequency in the frequency spectrum. For example, the specific frequency may be preset in accordance with the amplification characteristics of the amplifier circuit or set to the first frequency fp1 at which the strength in the frequency spectrum indicates the maximum value Pmax.

The second value V2 may be a second strength in a frequency range (also referred to as a third frequency range) A3 including the first frequency fp1 at which the strength indicates the maximum value Pmax in either the first or second frequency spectrum. As shown in, for example, FIG. 6B, the third frequency range A3 can be set to a predetermined width range with reference to the first frequency fp1 at which the strength in the frequency spectrum indicates the maximum value Pmax. FIG. 6B shows an example of the frequency spectrum with a curve Ln10 indicated with a bold dot-dash chain line. More specifically, the third frequency range A3 can be set to, for example, a frequency range lower than or equal to the first frequency fp1. In this case, as shown in FIG. 6B for example, the computation processor 30a determines the first frequency fp1 at which the signal strength indicates the maximum value Pmax in the frequency spectrum. The computation processor 30a then uses, for example, the second strength in the third frequency range A3 including the first frequency fp1 as the second value V2. If the second strength is the first maximum strength Pmax, the second strength can be easily obtained. Thus, the flow quantitative value ranging from a small to a large value and the flow calculation value Vc easily have an approximate proportional relation. Thus, the measurement device 1 can easily improve the measurement accuracy. In this case, as shown in FIG. 6B, the second strength is the maximum value Pmax. As shown in FIG. 6B, the computation processor 30a determines the maximum value Pmax of the signal strength in the frequency spectrum. The computation processor 30a then uses, for example, the maximum value Pmax as the second value V2.

The second value V2 may also be a value of the strength calculated by one or more calculations such as multiplication by a coefficient or exponentiation on the above-described third integrated value or the second strength. In other words, a value of one strength selected from the above-described third integrated value and the second strength may be used as an example of the second value V2. In another example, a value of at least one strength selected from the above-described third integrated value and the second strength may be used as an example of the second value V2. In such structures as well, the flow quantitative value ranging from a small to a large value and the flow calculation value Vc have an approximate proportional relation. Thus, the measurement device 1 can improve the measurement accuracy. The sum of the above-described third integrated value and the second strength may be used as an example of the second value V2.

Calculation of Flow Quantitative Value

The computation processor 30a can calculate a quantitative value (flow quantitative value) indicating the flow state of the fluid 2b based on, for example, the calculated flow calculation value Vc. For example, the computation processor 30a can calculate the quantitative value (flow quantitative value) on the flow of the fluid 2b based on the calculated flow calculation value Vc and prepared calibration data (also referred to as a calibration curve). If, for example, the calibration data on the flow rate of the fluid 2b is prepared in advance, the flow rate of the fluid 2b can be calculated based on the flow calculation value Vc and the calibration curve of the flow rate serving as the flow quantitative value. If, for example, the calibration data on the flow velocity of the fluid 2b is prepared in advance, the flow velocity of the fluid 2b can be calculated based on the flow calculation value Vc and the calibration curve of the flow velocity serving as the flow quantitative value. Thus, at least one of the flow rate or the flow velocity of the fluid 2b can be calculated. As described above, for example, the flow calculation value Vc tends to increase in proportion to the increase in the flow quantitative value ranging from a small to a large value. Thus, the measurement device 1 can improve the measurement accuracy.

For example, the calibration data may be stored in the storage 30b in advance before the flow quantitative value of the fluid 2b is measured. The calibration data may be stored in the form of, for example, a functional formula or a table.

The calibration data can be prepared by, for example, the measurement device 1 calculating the flow calculation value Vc of the fluid 2b flowing through the flow passage component 2a at a known flow quantitative value. The calculation of the flow calculation value Vc performed by the measurement device 1 includes the light emitter 11 irradiating the irradiation target 2 with the light L1, the light receiver 12 receiving the coherent light L2 including light scattered by the irradiation target 2, and the computation processor 30a calculating the flow calculation value Vc. For example, the measurement device 1 calculates the flow calculation value Vc of the fluid 2b flowing through the flow passage component 2a at a known flow quantitative value, and derives calibration data based on the relationship between the known flow quantitative value and the calculated flow calculation value Vc. More specifically, for example, an operation expression (calibration curve) including the flow calculation value Vc as a parameter is derived as calibration data.

For example, the calibration curve is expressed by formula (5) including the flow quantitative value denoted with y, the flow calculation value denoted with x, a coefficient a, and a constant b.

$$y = ax + b \qquad (5)$$

For example, if a flow calculation value y1 of the fluid 2b flowing through the flow passage component 2a at a known flow quantitative value x1 is calculated, and a flow calculation value y2 of the fluid 2b flowing through the flow passage component 2a at a known flow quantitative value x2 is calculated, formula (6) and formula (7) are obtained:

$$y1 = ax1 + b \qquad (6)$$

$$y2 = ax2 + b \qquad (7)$$

The coefficient a and the constant b are calculated using formula (6) and formula (7). The calculated coefficient a and the calculated constant b are substituted in formula (5), and thus the calibration data indicating the calibration curve can be obtained.

The functional formula indicating the calibration curve may be, for example, expressed in a polynomial expression including an n-th order (where n is a natural number of greater than or equal to 2) term where the flow quantitative value is denoted with y and the flow calculation value is a variable x. The functional formula indicating the calibration curve may include, for example, at least one term selected from the term of logarithm and a term of exponentiation of a variable x serving as the flow calculation value.

1-2. Operation of Measurement Device

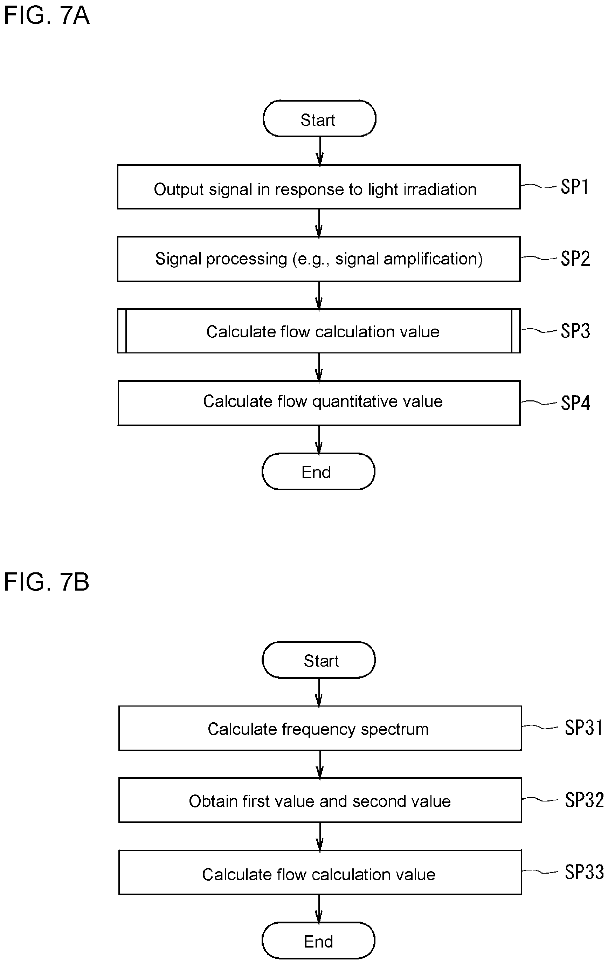

The operation of the measurement device 1 will now be described using an example. FIGs. 7A and 7B are flowcharts showing examples of the operation of the measurement device 1. The operation can be performed by, for example, the computation processor 30a executing the program PG1 and the controller 30 controlling the operation of the measurement device 1. The flow quantitative value indicating the flow state of the fluid 2b can be calculated by performing steps SP1 to SP4 in FIG. 7A.

Step SP1 in FIG. 7A is a process (also referred to as a first process) in which, while the light emitter 11 irradiates the irradiation target 2 having the internal space 2i through which the fluid 2b flows with light, the light receiver 12 receives the coherent light L2 including light scattered by the irradiation target 2 and outputs a signal corresponding to the intensity of the coherent light L2.

Step SP2 is a process (also referred to as a second process) in which the amplifier 20a in the signal processor 20 amplifies the signal output from the light receiver 12 in step SP1. Instead of amplification of the signal strength, the signal processor 20 may perform other operations on the signal output from the light receiver 12, such as AD conversion. The signal that has undergone the operation from the signal processor 20 is input into the controller 30. Thus, the controller 30 obtains information on the temporal change in the signal corresponding to the intensity of the coherent light L2 from the irradiation target 2.

Step SP3 is a process (also referred to as a third process) in which the computation processor 30a in the controller 30 calculates the flow calculation value Vc on the flow state of the fluid 2b in the internal space 2i of the irradiation target 2 based on the temporal strength change in the signal amplified by the amplifier 20a in the signal processor 20 in step SP2. Step SP3 includes steps SP31 to SP33 in FIG. 7B.

In step SP31, the computation processor 30a calculates the distribution (first frequency spectrum) of the signal strength for each frequency with respect to the temporal strength change in the signal amplified by the amplifier 20a in step SP2.

In step SP32, the computation processor 30a obtains the first value V1 on the frequency based on the first frequency spectrum calculated in step SP31 and obtains the second value V2 on the frequency based on the first frequency spectrum calculated in step SP31.

In step SP33, the computation processor 30a calculates the flow calculation value Vc on the flow state of the fluid 2b in the internal space 2i of the irradiation target 2 with a computation including division using the first and second values V1 and V2 obtained in step SP32. If the flow calculation value Vc is calculated by dividing the first value V1 by the second value V2, the flow quantitative value ranging from a small to a large value and the flow calculation value Vc easily have an approximate proportional relation. Thus, the measurement device 1 can easily improve the measurement accuracy.

In step SP4, the computation processor 30a calculates the flow quantitative value indicating the flow state of the fluid 2b in the internal space 2i of the irradiation target 2 based on the flow calculation value Vc calculated in step SP3. The flow quantitative value includes at least one of the flow rate or the flow velocity of the fluid 2b.

## 1-3. Overview of First Embodiment

The measurement device 1 according to the first embodiment calculates, for example, the flow calculation value Vc on the flow state of the fluid 2b in the internal space 2i of the irradiation target 2 with a computation including division using the first value V1 on the frequency having an increase rate decreasing, and the second value V2 on the strength decreasing with attenuation of the amplification factor with respect to the increase in the frequency in the amplifier 20a based on the first frequency spectrum for the signal corresponding to the intensity of the coherent light L2. The decrease in the increase rate of the first value V1 in accordance with the increase in the frequency and the decrease in the second value in accordance with the increase in the frequency cancel each other, and the flow calculation value Vc thus tends to increase in proportion to the increase in the flow quantitative value ranging from a small to a large value. In other words, the relationship between the flow quantitative value ranging from a small to a large value and the flow calculation value Vc have an approximate proportional relation. Thus, the measurement device 1 can improve the measurement accuracy.

## 2. Second Embodiment

The controller 30 may perform, with the computation processor 30a, a computation different from that according to the first embodiment for quantitatively measuring the flow state of the fluid 2b based on an electric signal output from the light receiver 12.

## 2-1. Computation with Computation Processor

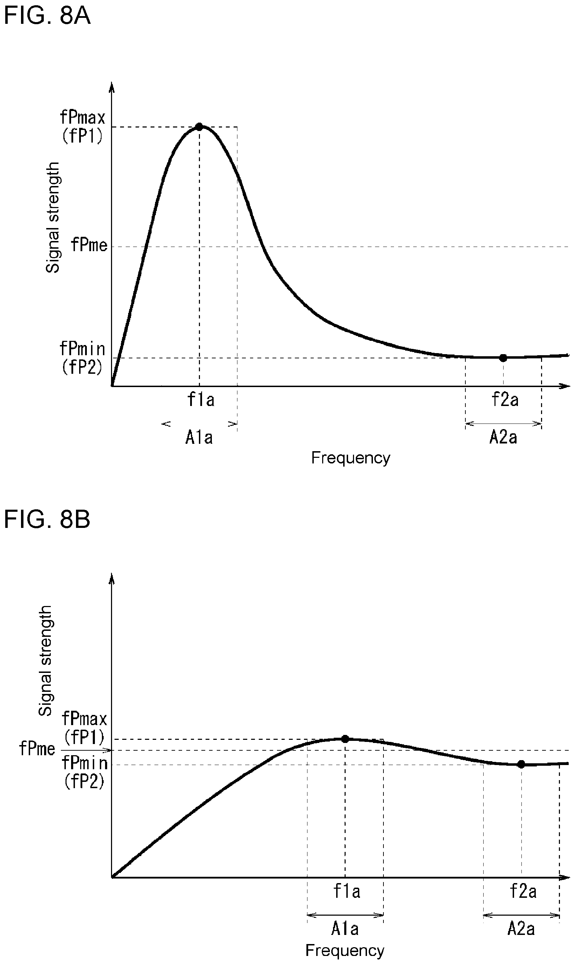

The computation processor 30a can calculate, for example, the distribution of the signal strength for each frequency (also referred to as a first distribution) with respect to changes in a signal output from the light receiver 12 over time (temporal strength change). The computation processor 30a can perform an analysis using, for example, a computation such as a Fourier transform for the temporal strength change in a signal output from the light receiver 12, and thus can calculate the power spectrum (also referred to as a frequency spectrum) indicating the distribution (first distribution) of the signal strength for each frequency for the coherent light L2 received by the light receiver 12. The frequency range in the frequency spectrum can be set, for example, based on the sampling rate in the AD conversion circuit. The computation processor 30a may calculate the first distribution by performing various computations on the frequency spectrum obtained by the analysis including a computation such as a Fourier transform with respect to the temporal strength change in a signal output from the light receiver 12. Examples of the various computations may include a weighting computation on the frequency spectrum with a frequency. The computation processor 30a can calculate, for example, the weighted frequency spectrum as the first distribution by weighting the strength for each frequency in the frequency spectrum with the corresponding frequency. For the fluid 2b with a relatively small flow rate or flow velocity, the computation processor 30a can calculate, for example, the first distribution (frequency spectrum) indicated with a bold line in FIG. 8A. For the fluid 2b with a relatively large flow rate or flow velocity, the computation processor 30a can calculate, for example, the first distribution (frequency spectrum) indicated with a bold line in FIG. 8B.

The computation processor 30a can determine, for example, the maximum value (also referred to as a maximum signal strength) fPmax of the signal strength in the first distribution as a first signal strength fP1. The computation processor 30a can determine, for example, the frequency (also referred to as a first A-frequency) f1a indicating the first signal strength fP1 in the first distribution. The computation processor 30a can also determine, for example, the minimum value (also referred to as a minimum signal strength) fPmin of the signal strength in the frequency range higher than the first A-frequency f1a in the first distribution as a second signal strength fP2. FIGs. 8A and 8B show the frequency (also referred to as a second A-frequency) f2a indicating the minimum signal strength fPmin in the first distribution. The computation processor 30a can calculate, for example, the calculation value (flow calculation value) Vc on the flow state of the fluid 2b flowing through the internal space 2i of the irradiation target 2 by dividing the second signal strength fP2 by the first signal strength fP1. In other words, for example, the flow calculation value Vc is calculated with formula (8), the first signal strength fP1, and the second signal strength fP2. Examples of the flow state include at least one of the flow rate or the flow velocity.

$$Vc = fP2/fP1 \qquad (8)$$

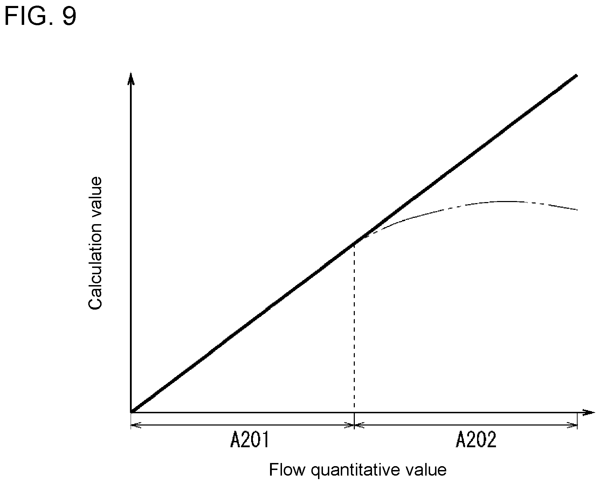

In this example, the measurement device 1 calculates the flow calculation value Vc while the quantitative value (flow quantitative value) on the flow state of the fluid 2b flowing through a transparent tube serving as the flow passage component 2a is set with, for example, a pump. In this structure, as shown in FIG. 9, the flow calculation value Vc tends to increase in proportion to the increase in the flow quantitative value.

If the flow rate set value serving as the flow quantitative value is changed from the relatively small value Qa to the relatively large value Qb, for example, the frequency at which the signal strength in the frequency spectrum has a maximum value shifts toward a higher frequency, as shown in FIG. 18. If the flow quantitative value is changed from the relatively small value to the relatively large value, for example, the frequency at which the signal strength in the frequency spectrum has a maximum value shifts toward a higher frequency, as shown in FIGs. 8A and 8B. In the frequency spectrum, in accordance with the increase in the flow quantitative value, the maximum signal strength fPmax tends to decrease and the minimum signal strength fPmin tends to increase. If the flow calculation value Vc using formula (8) described above is calculated with such tendencies, the flow calculation value Vc tends to increase in proportion to the increase in the flow quantitative value as indicated with a bold line in FIG. 9. In other words, the flow quantitative value ranging from a relatively low range to a relatively high range and the flow calculation value may have a linear relation. FIG. 9 shows the relationship between the flow rate set value and the flow rate calculation value shown in FIG. 19 with a two-dot chain line for comparison.

Instead of directly dividing the second signal strength fP2 by the first signal strength fP1, the computation for calculating the flow calculation value Vc may be, for example, division of a value (also referred to as a second A-value) of the second signal strength fP2 by a value (also referred to as a first A-value) of the first signal strength fP1. The first A-value can be calculated by, for example, performing at least one calculation such as multiplication by a coefficient or exponentiation on the first signal strength fP1. The second A-value can be calculated by, for example, performing at least one calculation such as multiplication by a coefficient or exponentiation on the second signal strength fP2. The first signal strength fP1 may be directly used as the first A-value, or the second signal strength fP2 may be directly used as the second A-value.

2-2. Operation of Measurement Device

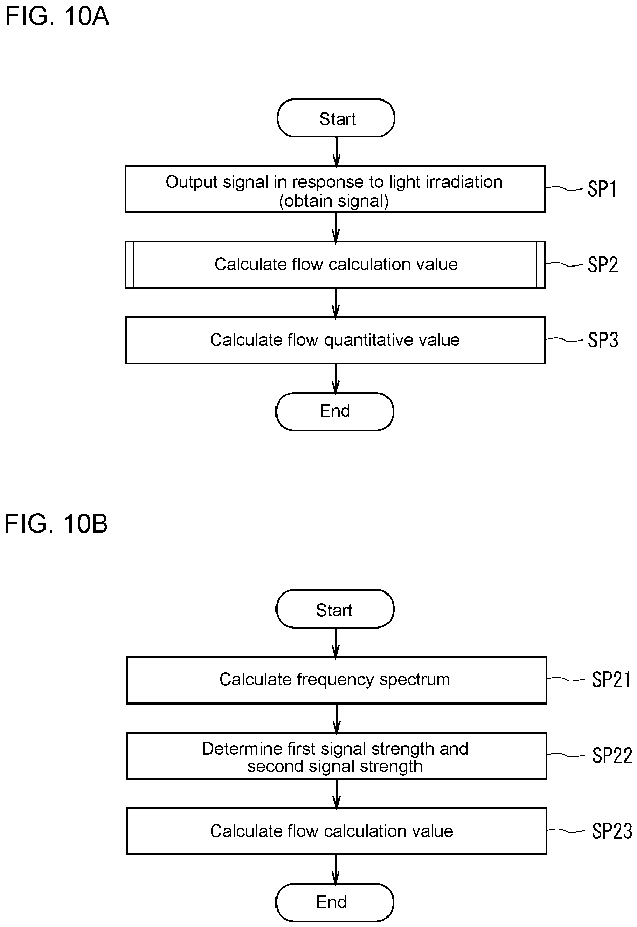

The operation of the measurement device 1 according to the second embodiment will now be described using an example. FIGs. 10A and 10B are flowcharts showing an example operation of the measurement device 1 according to the second embodiment. This operation can be performed by, for example, the computation processor 30a executing the program PG1 and the controller 30 controlling the operation of the measurement device 1. Performing steps SP1 to SP3 in FIG. 10A enables a calculation of the flow quantitative value indicating the flow state of the fluid 2b.

Step SP1 in FIG. 10 is a process (also referred to as a first A-process) in which, while the light emitter 11 irradiates the irradiation target 2 having the internal space 2i through which the fluid 2b flows with light, the light receiver 12 receives the coherent light L2 including the light scattered by the irradiation target 2 and outputs a signal corresponding to the intensity of the coherent light L2. The signal output from the light receiver 12 is input into the controller 30 after undergoing, for example, amplification and AD conversion with the signal processor 20. Thus, the controller 30 obtains information on the temporal change in a signal corresponding to the intensity of the coherent light L2 from the irradiation target 2.

Step SP2 is a process (also referred to as a second A-process) in which the computation processor 30a calculates the flow calculation value Vc on the flow state of the fluid 2b in the internal space 2i of the irradiation target 2 based on the temporal strength change in the signal output from the light receiver 12 in step SP1. Step SP2 includes steps SP21 to SP23 in FIG. 10B.

In step SP21, the computation processor 30a calculates the distribution (first distribution) of the signal strength for each frequency with respect to the temporal strength change in the signal output from the light receiver 12 in step SP1. The first distribution corresponds to the frequency spectrum.

In step SP22, the computation processor 30a determines the maximum signal strength fPmax serving as the first signal strength fP1 in the first distribution and determines the minimum signal strength fPmin serving as the second signal strength fP2 in the frequency range higher than the first A-frequency fla indicating the first signal strength fP1 in the first distribution.

In step SP23, the computation processor 30a calculates the flow calculation value Vc on the flow state of the fluid 2b in the internal space 2i of the irradiation target 2 by dividing the minimum signal strength fPmin serving as the second signal strength fP2 by the maximum signal strength fPmax serving as the first signal strength fP1.

In step SP3, the computation processor 30a calculates a flow quantitative value indicating the flow state of the fluid 2b in the internal space 2i of the irradiation target 2 based on the flow calculation value Vc calculated in step SP2. The flow quantitative value includes at least one of the flow rate or the flow velocity of the fluid 2b.

2-3. Overview of Second Embodiment

The measurement device 1 according to the second embodiment calculates, for example, the flow calculation value Vc by dividing the minimum signal strength fPmin in the frequency range higher than the first A-frequency fIa indicating the maximum signal strength fPmax by the maximum signal strength fPmax for the first distribution of the signal strength for each frequency with respect to the temporal change in the signal strength in the coherent light L2. Thus, the flow calculation value Vc tends to increase in proportion to the increase in the flow quantitative value ranging from a small to a large value. In other words, the flow quantitative value ranging from a small to a large value and the flow calculation value Vc have an approximate proportional relation. Thus, the measurement device 1 can improve the measurement accuracy.

2-4. Modification of Second Embodiment

In the second embodiment, the computation processor 30a may calculate, for example, the distribution (first distribution) of the signal strength for each frequency to reduce a noise component with respect to the temporal strength change in a signal output from the light receiver 12. The noise component can be reduced with, for example, information on the temporal change in the signal strength corresponding to the intensity of the coherent light L2 obtained while the fluid 2b stops flowing in the internal space 2i of the irradiation target 2.

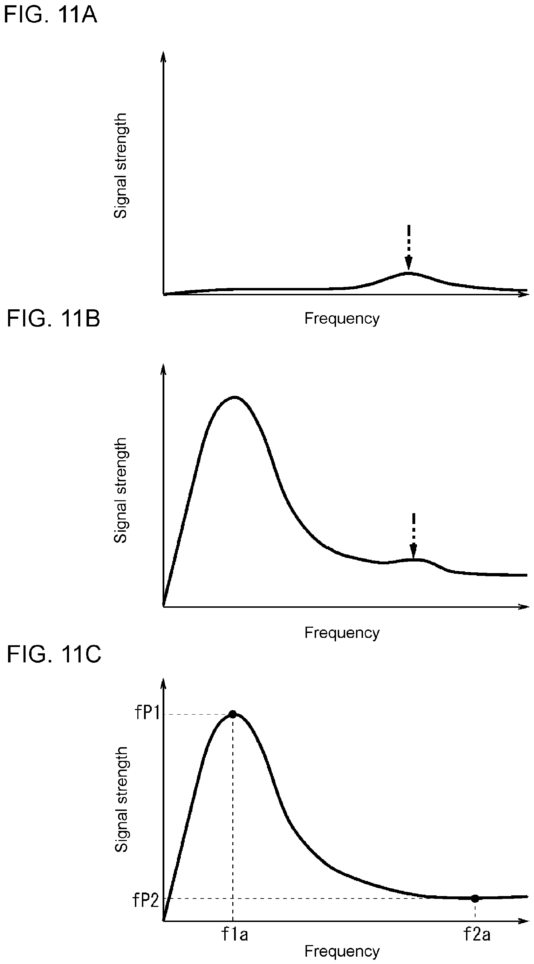

More specifically, for example, the light emitter 11 irradiates the irradiation target 2 having the internal space 2i in which the fluid 2b stops flowing with the light L1. The flow of the fluid 2b in the internal space 2i of the irradiation target 2 can be stopped by setting the quantitative value (flow quantitative value) on the flow state of the fluid 2b flowing through the flow passage component 2a such as a transparent tube with, for example, a pump. The light receiver 12 receives the coherent light L2 including, for example, the light scattered by the irradiation target 2, and outputs a signal corresponding to the intensity of the coherent light L2. The computation processor 30a calculates the distribution (also referred to as a second distribution) of the signal strength for each frequency with respect to the temporal strength change in the signal output from the light receiver 12. In other words, the computation processor 30a can calculate the second distribution with respect to the temporal change in the signal strength corresponding to the intensity of the coherent light output in response to the light receiver 12 receiving the coherent light L2 including the light scattered by the irradiation target 2 if the light emitter 11 irradiates the irradiation target 2 having the internal space 2i in which the fluid 2b stops flowing with the light L1. The computation processor 30a can calculate, for example, the frequency spectrum serving as the second distribution indicated with a bold line in FIG. 11A. The second distribution corresponds to, for example, the frequency spectrum with respect to the temporal change in the signal strength corresponding to the intensity of the coherent light caused by the external environment out of the fluid 2b, rather than caused by the flow of the fluid 2b. The second distribution can represent a noise component caused by the external environment. Examples of the noise caused by the external environment can include a noise caused by vibrations of the flow passage component 2a such as a transparent tube and an electromagnetic noise caused by the signal processor 20 and the controller 30. In the example in FIG. 11A, a portion indicated with a bold two-dot-chain-line arrow shows a noticeable effect of the noise caused by the external environment.

The computation processor 30a can also calculate, for example, the distribution (also referred to as a third distribution) of the signal strength for each frequency with respect to the temporal change in the signal strength corresponding to the intensity of the coherent light output in response to the light receiver 12 receiving the coherent light L2 including the light scattered by the irradiation target 2 if the light emitter 11 irradiates the irradiation target 2 having the internal space 2i in which the fluid 2b flows with the light L1. The computation processor 30a can calculate the frequency spectrum serving as the third distribution indicated with a bold line in FIG. 11B. In the example in FIG. 11B, a portion indicated with a bold two-dot-chain-line arrow shows a noticeable effect of the noise caused by the external environment.

The computation processor 30a can calculate, for example, the first distribution with reduced noise components by performing a computation, with the second distribution, of reducing the noise components in the signal strength in the third distribution for each frequency. The computation processor 30a can calculate, for example, the frequency spectrum serving as the first distribution indicated with a bold line in FIG. 11C. Reducing the effects of the noise caused by the external environment on measurement of the flow quantitative value can improve the measurement accuracy in a quantitative measurement on the flow state of the fluid 2b. Noise is more likely to be caused by the external environment if the fluid 2b flows in the internal space 2i of the irradiation target 2 than if the fluid 2b stops flowing in the internal space 2i of the irradiation target 2. Using the division of the signal strength of the third distribution by the signal strength of the second distribution for each frequency as a computation for reducing the noise components can reduce the effect of the noise caused by the external environment on measurement of the flow quantitative value. Thus, the measurement device 1 can improve the measurement accuracy.

Examples of a computation for reducing the noise components may include, for example, dividing a value (also referred to as a fourth A-value) of the signal strength of the third distribution by a value (also referred to as a third A-value) of the signal strength of the second distribution for each frequency. The third A-value can be calculated by performing, for example, at least one calculation such as multiplication by a coefficient or exponentiation on the signal strength of the second distribution. The fourth A-value can be calculated by performing, for example, at least one calculation such as multiplication by a coefficient or exponentiation on the signal strength of the third distribution. The signal strength of the second distribution may be directly used as the third A-value, or the signal strength of the third distribution may be directly used as the fourth A-value. Subtracting the signal strength of the second distribution from the signal strength of the third distribution for each frequency may be used as the computation for reducing the noise components. Subtracting the third A-value of the signal strength of the second distribution from the fourth A-value of the signal strength of the third distribution for each frequency may be used as the computation for reducing the noise components.

3. Other Embodiments

The present disclosure is not limited to the first and second embodiments and may be changed or modified in various manners without departing from the spirit and scope of the present disclosure.

3-1. Third Embodiment

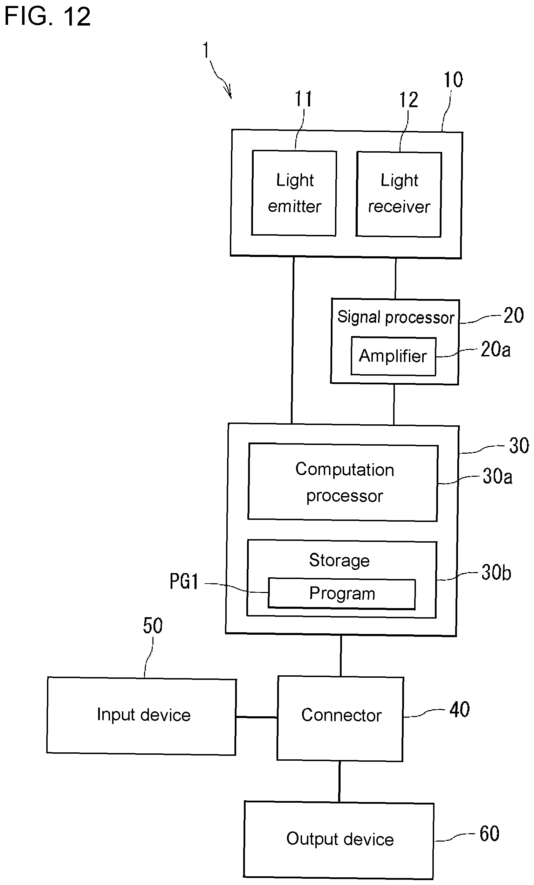

As shown in FIG. 12, the measurement device 1 according to each of the embodiments may include, for example, an input device 50 or an output device 60.

The input device 50 is connectable to the controller 30 through the connector 40. In response to, for example, the operation of a user, the input device 50 can input various conditions (also referred to as measurement conditions) on a measurement of the flow quantitative value in the measurement device 1 into the controller 30. Examples of the measurement conditions include the frequency range in the frequency spectrum calculated by the computation processor 30a. Examples of the input device 50 include an operation portion such as a keyboard, a mouse, a touchscreen, and a switch, and a microphone enabling voice input. The input device 50 enables a user to easily set intended measurement conditions. Thus, the measurement device 1 can enhance user convenience. The measurement conditions may also include, for example, the light quantity of the light L1 emitted from the light emitter 11, a cycle in which the light receiver 12 outputs a signal, the sampling rate in AD conversion, an operation expression on calibration data and a coefficient in this operation expression, and a coefficient and an exponent in division or subtraction. The input device 50 may also enable input of various sets of information on the fluid 2b such as the viscosity, concentration, or the size of a scatterer in the fluid 2b.

The output device 60 is connectable to, for example, the controller 30 through the connector 40. The output device 60 may include a display that visibly outputs various sets of information on measurements of the flow quantitative value or a speaker that audibly outputs various sets of information on measurements of the flow quantitative value. Examples of the display include a liquid crystal display and a touchscreen. If the input device 50 includes a touchscreen, the displays of the input device 50 and the output device 60 may be formed from a single touchscreen. The measurement device 1 with this structure includes fewer components, is downsized, and facilitates manufacture. A display that can visibly display measurement conditions, the frequency spectrum, or the flow calculation value or flow quantitative value serving as the measurement enables a user to easily view the various sets of information on measurements of the flow quantitative value. For example, the display may allow a user to change the output form of various sets of information in the output device 60 through the input device 50. The change in the output form may include, for example, a change in the display form and switching of displayed information. The display thus allows a user to easily view the various sets of information on measurements of the flow quantitative value. Thus, the measurement device 1 can enhance user convenience. 3-2. Fourth Embodiment

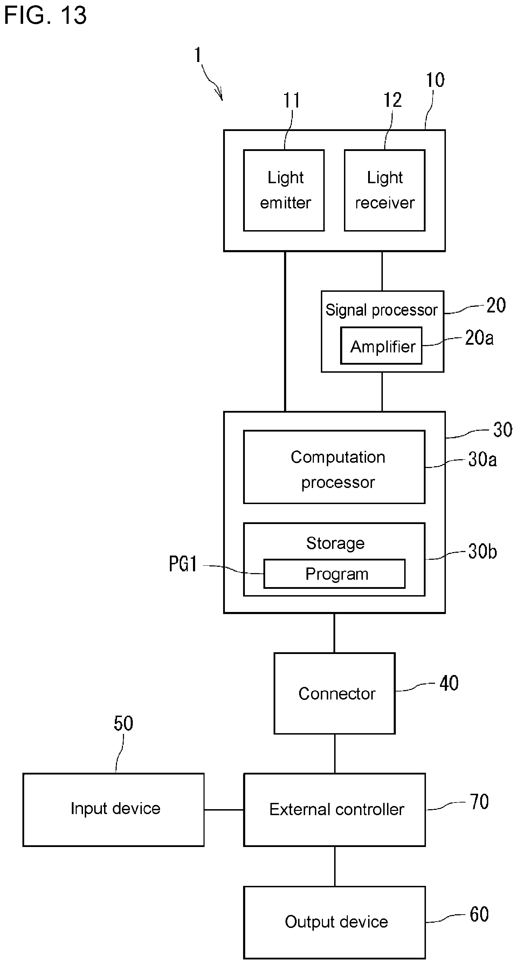

As shown in FIG. 13, the measurement device 1 according to each embodiment may also include, for example, an external controller 70. The external controller 70 may include, for example, a computer such as a microcomputer.

The external controller 70 holds measurement conditions such as the light quantity of the light L1, a cycle in which the light receiver 12 outputs a signal, and the sampling rate in AD conversion. These measurement conditions may be input into the controller 30. Thus, the processes to be performed by the computation processor 30a can be reduced, and thus the controller 30 can improve the processing speed. The measurement conditions include, for example, the same conditions as the various conditions on the measurement of the flow quantitative value in the measurement device 1 that can be input by the input device 50.

The external controller 70 may control the input device 50 and the output device 60. This structure reduces units having various functions (also referred to as functional units) controlled by the controller 30, and thus can improve the processing speed of the controller 30. The external controller 70 may include, for example, various other functional units including multiple electronic components. Examples of the various other functional units include a pressure gauge and a thermometer. Thus, the measurement device 1 can enhance design flexibility and user convenience.

The external controller 70, the controller 30, the input device 50, and the output device 60 may communicate with one another with wires or wirelessly. The controller 30 and the external controller 70 communicate with each other in accordance with any telecommunications standard. Such telecommunications standards include Inter-Integrated Circuit (IIC), the Serial Peripheral Interface (SPI), and a universal asynchronous receiver transmitter (UART).

The sensor 10, the signal processor 20, and the external controller 70 may directly communicate with one another. In this case, the measurement device 1 may eliminate the controller 30, and the external controller 70 may serve as the controller 30. For example, the sensor 10, the signal processor 20, and the external controller 70 communicate directly with one another to eliminate delays of signals between the controller 30 and the external controller 70. The measurement device 1 can thus improve the processing speed and enhance user convenience.

3-3. Fifth Embodiment

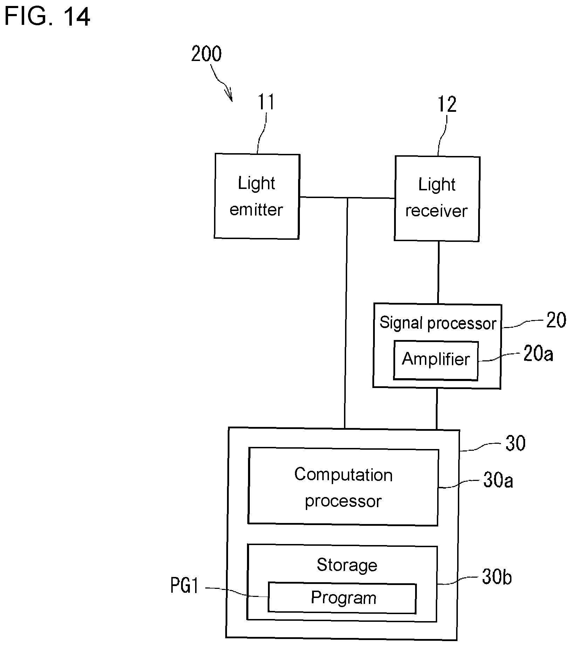

In each of the above embodiments, a measurement system 200 may include all the components or at least two components of the measurement device 1 connected to allow communication between them. As shown in FIG. 14, for example, the measurement system 200 according to the fifth embodiment includes the light emitter 11, the light receiver 12, the signal processor 20 including the amplifier 20a, and the controller 30 including the computation processor 30a. In the example shown in FIG. 14, the light emitter 11 and the light receiver 12, the light emitter 11 and the controller 30, the light receiver 12 and the signal processor 20, and the signal processor 20 and the controller 30 are connected to allow communication between them. The signal processor 20 according to the second embodiment may eliminate the amplifier 20a.

4. Others

In the first and third to fifth embodiments, the computation processor 30a may calculate, for example, the distribution of the signal strength for each frequency (first frequency spectrum) to reduce noise components with respect to the temporal strength change in the signal amplified by the amplifier 20a in the signal processor 20. The noise components can be reduced using information on the temporal change in the signal strength corresponding to the intensity of the coherent light L2 obtained while the fluid 2b stops flowing in the internal space 2i of the irradiation target 2.

In the first and third to fifth embodiments, a computation including division using the first and second values V1 and V2 performed by the computation processor 30a may be, for example, a computation including division of the second value V2 by the first value V1. This computation yields the flow calculation value Vc for which the reduction of the increase rate of the first value V1 in accordance with an increase in the flow quantitative value cancels the reduction of the second value V2 in accordance with an increase in the flow quantitative value.

In the first and third to fifth embodiments, the frequency shift amount Δf serving as the first value V1 may be an inverse of inclination indicating the ratio of a change in the signal strength to the change in frequency in the first or second frequency spectrum.

In the second to fifth embodiments, the first signal strength fP1 in the first distribution may be any signal strength in the frequency range (also referred to as a first A-frequency range) A1a including the first A-frequency f1a indicating the maximum signal strength fPmax in the first distribution. The second signal strength fP2 in the first distribution may be any signal strength in the frequency range (also referred to as a second A-frequency range) A2a including the second A-frequency f2a indicating the minimum signal strength fPmin in the frequency range higher than the first A-frequency range A1a in the first distribution. In such a structure as well, the flow quantitative value ranging from a small to a large value and the flow calculation value Vc have an approximate proportional relation.

As shown in, for example, FIGs. 8A and 8B, the first A-frequency range A1a can be set to a predetermined width range with reference to the first A-frequency f1a. As shown in, for example, FIGs. 8A and 8B, the second A-frequency range A2a can be set to a predetermined width range with reference to the second A-frequency f2a. More specifically, the first A-frequency range A1a can be set to a predetermined width range having the first A-frequency f1a at the center. The second A-frequency range A2a can be set to a predetermined width range having the second A-frequency f2a at the center. The first and second A-frequency ranges A1a and A2a may be any ranges that do not overlap each other. For example, the first and second A-frequency ranges A1a and A2a may be set on both sides of a middle frequency between the first and second A-frequencies f1a and f2a. The first and second A-frequency ranges A1a and A2a may be adjacent to or separate from each other. The first signal strength fP1 may be, for example, larger than or equal to 0.8 or 0.9 times the maximum signal strength fPmax. The second signal strength fP2 may be, for example, smaller than or equal to 1.2 or 1.1 times the minimum signal strength fPmin.

The first signal strength fP1 in the first distribution may be, for example, larger than a mean value fPme of the maximum and minimum signal strengths fPmax and fPmin. The second signal strength fP2 in the first distribution may be, for example, smaller than the mean value fPme of the maximum and minimum signal strengths fPmax and fPmin. In such a structure as well, the flow quantitative value ranging from a small to a large value and the flow calculation value Vc have an approximate proportional relation. The first signal strength fP1 may be larger than or equal to 0.8 or 0.9 times the maximum signal strength fPmax. The second signal strength fP2 may be smaller than or equal to 1.2 or 1.1 times the minimum signal strength fPmin.

As in each embodiment, for example, if the maximum signal strength fPmax is the first signal strength fP1 and the minimum signal strength fPmin is the second signal strength fP2, the flow quantitative value ranging from a small to a large value and the flow calculation value Vc easily have an approximate proportional relation. Thus, the measurement device 1 can easily improve the measurement accuracy.

In each embodiment, the computation processor 30a may eliminate a calculation of the flow quantitative value indicating the flow state of the fluid 2b based on the flow calculation value Vc on the flow of the fluid 2b. The structure also enables a user to monitor a change in the flow state of the fluid 2b based on the change in the flow calculation value Vc. Thus, the measurement device 1 that quantitatively measures the flow state of the fluid 2b can improve the measurement accuracy.

In each embodiment, at least one of the functions of the computation processor 30a may be implemented in hardware such as a dedicated electronic circuit.

The components described in the above embodiments may be entirely or partially combined as appropriate unless any contradiction arises.

Reference Signs List

| | |
|---|---|
| 1 | measurement device |
| 2 | irradiation target |
| 2a | flow passage component |
| 2b | fluid |
| 2i | internal space |
| 10 | sensor |
| 11 | light emitter |
| 12 | light receiver |
| 20 | signal processor |
| 30 | controller |
| 30a | computation processor |
| 30b | storage |
| 70 | external controller |
| 200 | measurement system |
| A1 | first frequency range |
| A2 | second frequency range |
| A3 | third frequency range |
| A1a | first A-frequency range |
| A2a | second A-frequency range |
| Ar1 | first integrated value |
| Ar2 | second integrated value |
| L1 | light |

| | |
|---|---|
| L2 | coherent light |
| PG1 | program |
| Pmax | first maximum strength (maximum value) |
| V1 | first value |
| V2 | second value |
| Vc | flow calculation value |
| f1a | first A-frequency |
| f2a | second A-frequency |
| fm | mean frequency |
| fo | frequency |
| fp1 | first frequency |
| fp2 | second frequency |
| fP1 | first signal strength |
| fP2 | second signal strength |
| fPmax | maximum signal strength |
| fPmin | minimum signal strength |

**Claims**

1. A measurement device, comprising:

a light emitter configured to irradiate, with light, an irradiation target having an internal space through which a fluid flows;
a light receiver configured to receive coherent light including light scattered by the irradiation target and to output a signal corresponding to intensity of the coherent light;
an amplifier configured to amplify the signal output from the light receiver; and
a computation processor configured to calculate a first frequency spectrum of signal strength for each frequency with respect to a temporal strength change in the signal amplified by the amplifier, and to calculate a calculation value on a flow state of the fluid with a computation including division using a first value of a frequency based on the first frequency spectrum and a second value of strength based on the first frequency spectrum.

2. The measurement device according to claim 1, wherein
the division includes dividing the first value by the second value.

3. The measurement device according to claim 1 or claim 2, wherein

the first value includes a value of at least one frequency selected from the group consisting of a frequency in the first frequency spectrum or a second frequency spectrum calculated by performing a computation on the first frequency spectrum at a boundary at which a first integrated value and a second integrated value have a predetermined ratio, the first integrated value is obtained by integration on strength of a lower frequency, and the second integrated value is obtained by integration on strength of a higher frequency,

a frequency of first strength in a first frequency range including a first frequency having strength indicating a maximum value,

a frequency of first inclination in a second frequency range including a second frequency having an absolute value of inclination of a strength change indicating a minimum value, and

a frequency shift amount between a frequency of light with which the light emitter irradiates the irradiation target and a frequency of the coherent light.

4. The measurement device according to claim 3, wherein
the first strength is the maximum value.

5. The measurement device according to claim 3 or claim 4, wherein
the absolute value for the first inclination is the minimum value.

6. The measurement device according to claim 3, wherein
the shift amount includes a mean frequency for the first frequency spectrum or a frequency range width for the first frequency spectrum indicating strength higher than or equal to a specific value.

7. The measurement device according to any one of claims 1 to 6, wherein
the second value includes a value of at least one strength selected from the group consisting of

   a third integrated value obtained by integration on the first frequency spectrum or a second frequency spectrum calculated by performing a computation on the first frequency spectrum, and
   second strength in a third frequency range including a first frequency having strength indicating a maximum value.

8. The measurement device according to claim 7, wherein
the second strength is the maximum value.

9. A measurement device, comprising:

   a light emitter configured to irradiate, with light, an irradiation target having an internal space through which a fluid flows;
   a light receiver configured to receive coherent light including light scattered by the irradiation target, and to output a signal corresponding to intensity of the coherent light; and
a computation processor configured to calculate a first distribution of signal strength for each frequency with respect to a temporal strength change in the signal output from the light receiver, and to calculate a calculation value on a flow state of the fluid with a computation including dividing a second A-value of second signal strength in a second A-frequency range by a first A-value of first signal strength in a first A-frequency range including a first A-frequency having signal strength indicating a maximum value in the first distribution, the second A-frequency range being included in a frequency range higher than the first A-frequency range and including a second A-frequency having signal strength indicating a minimum value in the first distribution.

10. The measurement device according to claim 9, wherein

   the first signal strength is higher than a mean value of the maximum value and the minimum value in the first distribution, and
   the second signal strength is smaller than the mean value in the first distribution.

11. The measurement device according to claim 9 or claim 10, wherein

   the first signal strength is the maximum value, and
   the second signal strength is the minimum value.

12. The measurement device according to any one of claims 9 to 11, wherein

   the computation processor calculates a second distribution of signal strength for each frequency with respect to a temporal strength change in the signal corresponding to intensity of the coherent light output in response to the light receiver receiving coherent light including light scattered by the irradiation target if the light emitter irradiates, with light, the irradiation target having the internal space through which the fluid stops flowing,
   calculates a third distribution of signal strength for each frequency with respect to a temporal strength change in the signal corresponding to intensity of the coherent light output in response to the light receiver receiving coherent light including light scattered by the irradiation target if the light emitter irradiates, with light, the irradiation target having the internal space through which the fluid flows, and
   calculates the first distribution by performing a computation with the second distribution for reducing a noise component in the signal strength of the third distribution for each frequency.

13. The measurement device according to claim 12, wherein
the computation for reducing the noise component includes dividing a fourth A-value of the signal strength of the third distribution by a third A-value of the signal strength of the second distribution for each frequency.

14. The measurement device according to any one of claim 1 to 13, wherein
the computation processor calculates a flow quantitative value indicating a flow state of the fluid based on the calculation value.

15. A measurement system, comprising:

a light emitter configured to irradiate, with light, an irradiation target having an internal space through which a fluid flows;
a light receiver configured to receive coherent light including light scattered by the irradiation target, and to output a signal corresponding to intensity of the coherent light;
an amplifier configured to amplify the signal output from the light receiver; and
a computation processor configured to calculate a first frequency spectrum of signal strength for each frequency with respect to a temporal strength change in the signal amplified by the amplifier, and to calculate a calculation value on a flow state of the fluid with a computation including division using a first value of a frequency based on the first frequency spectrum and a second value of strength based on the first frequency spectrum.

16. A measurement system, comprising:

a light emitter configured to irradiate, with light, an irradiation target having an internal space through which a fluid flows;
a light receiver configured to receive coherent light including light scattered by the irradiation target, and to output a signal corresponding to intensity of the coherent light; and
a computation processor configured to calculate a first distribution of signal strength for each frequency with respect to a temporal strength change in the signal output from the light receiver, and to calculate a calculation value on a flow state of the fluid with a computation including dividing a second A-value of second signal strength in a second A-frequency range by a first A-value of first signal strength in a first A-frequency range including a first A-frequency having signal strength indicating a maximum value in the first distribution, the second A-frequency range being included in a frequency range higher than the first A-frequency range and including a second A-frequency having signal strength indicating a minimum value in the first distribution.

17. A measurement method, comprising:

performing a first process of irradiating, with a light emitter, an irradiation target having an internal space through which a fluid flows with light, and receiving, with a light receiver, coherent light including light scattered by the irradiation target and outputting a signal corresponding to intensity of the coherent light;
performing a second process of amplifying, with an amplifier, the signal output from the light receiver in the first process; and
performing a third process of calculating, with a computation processor, a first frequency spectrum of signal strength for each frequency with respect to a temporal strength change in the signal amplified by the amplifier in the second process, and calculating a calculation value on a flow state of the fluid with a computation including division using a first value of a frequency based on the first frequency spectrum and a second value of strength based on the first frequency spectrum.

18. A measurement method, comprising:

performing a first A-process of irradiating, with a light emitter, an irradiation target having an internal space through which a fluid flows with light, and receiving, with a light receiver, coherent light including light scattered by the irradiation target and outputting a signal corresponding to intensity of the coherent light; and
performing a second A-process of calculating, with a computation processor, a first distribution of signal strength for each frequency with respect to a temporal strength change in the signal output from the light receiver in the first A-process, and calculating a calculation value on a flow state of the fluid with a computation including dividing a second A-value of second signal strength in a second A-frequency range by a first A-value of first signal strength in a first A-frequency range including a first A-frequency having signal strength indicating a maximum value in the first distribution, the second A-frequency range being included in a frequency range higher than the first A-frequency range and including a second A-frequency having signal strength indicating a minimum value in the first distribution.

**19.** A program executable by a processor included in a measurement device to cause the measurement device to function as the measurement device according to any one of claims 1 to 14.

FIG. 1

FIG. 2

FIG. 3

FIG. 4

FIG. 5A

FIG. 5B

FIG. 6A

FIG. 6B

## FIG. 7A

Start

Output signal in response to light irradiation — SP1

Signal processing (e.g., signal amplification) — SP2

Calculate flow calculation value — SP3

Calculate flow quantitative value — SP4

End

## FIG. 7B

Start

Calculate frequency spectrum — SP31

Obtain first value and second value — SP32

Calculate flow calculation value — SP33

End

FIG. 8A

FIG. 8B

# FIG. 9

## FIG. 10A

```
        ┌─────────────┐
        │    Start     │
        └──────┬──────┘
               │
               ▼
┌───────────────────────────────────────┐
│ Output signal in response to light     │ ── SP1
│ irradiation (obtain signal)            │
└──────────────────┬────────────────────┘
                   │
                   ▼
┌───────────────────────────────────────┐
║ Calculate flow calculation value       ║ ── SP2
└───────────────────────────────────────┘
                   │
                   ▼
┌───────────────────────────────────────┐
│ Calculate flow quantitative value      │ ── SP3
└──────────────────┬────────────────────┘
                   │
                   ▼
        ┌─────────────┐
        │     End      │
        └─────────────┘
```

## FIG. 10B

```
        ┌─────────────┐
        │    Start     │
        └──────┬──────┘
               │
               ▼
┌───────────────────────────────────────┐
│ Calculate frequency spectrum           │ ── SP21
└──────────────────┬────────────────────┘
                   │
                   ▼
┌───────────────────────────────────────┐
│ Determine first signal strength and    │ ── SP22
│ second signal strength                 │
└──────────────────┬────────────────────┘
                   │
                   ▼
┌───────────────────────────────────────┐
│ Calculate flow calculation value       │ ── SP23
└──────────────────┬────────────────────┘
                   │
                   ▼
        ┌─────────────┐
        │     End      │
        └─────────────┘
```

FIG. 11A

FIG. 11B

FIG. 11C

FIG. 12

Light emitter — 11

Light receiver — 12

— 10

Signal processor — 20

Amplifier — 20a

Computation processor — 30 / 30a

Storage

PG1 — Program — 30b

Input device — 50

Connector — 40

Output device — 60

FIG. 13

FIG. 14

200

11 Light emitter

12 Light receiver

Signal processor — 20
Amplifier — 20a

30
Computation processor — 30a

Storage — 30b
PG1 — Program

FIG. 15A

Ln101    Ln102    Ln103

Weighted signal strength
(signal strength P × frequency f)

fm1      fm2      fm3

Frequency

FIG. 15B

fm3

fm2

fm1

Calculation value
(mean frequency)

Q1       Q2       Q3

Set value

FIG. 16

FIG. 17A

FIG. 17B

FIG. 18

FIG. 19

**INTERNATIONAL SEARCH REPORT**

International application No.

PCT/JP2020/013640

**A. CLASSIFICATION OF SUBJECT MATTER**

G01F 1/66(2006.01)i; A61B 5/0285(2006.01)i; G01P 5/26(2006.01)i
FI: G01F1/66 103; A61B5/0285 H; G01P5/26 A

According to International Patent Classification (IPC) or to both national classification and IPC

**B. FIELDS SEARCHED**

Minimum documentation searched (classification system followed by classification symbols)
G01F1/66; A61B5/026-5/0295; G01P5/26

Documentation searched other than minimum documentation to the extent that such documents are included in the fields searched

| | |
|---|---|
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2000-210264 A (FUJI PHOTO FILM CO., LTD.) 02.08.2000 (2000-08-02) entire text, all drawings | 1–19 |
| A | JP 60-203235 A (FUJII, Hitoshi) 14.10.1985 (1985-10-14) page 3, upper right column, line 5 to page 3, lower left column, line 16, fig. 1-5 | 1–19 |
| A | JP 60-203236 A (FUJII, Hitoshi) 14.10.1985 (1985-10-14) page 3, upper left column, line 13 to page 3, lower left column, line 4, fig. 1-5 | 1–19 |
| A | WO 2019/035175 A1 (OLYMPUS CORP.) 21.02.2019 (2019-02-21) entire text, all drawings | 1–8, 14–15, 17, 19 |

☐ Further documents are listed in the continuation of Box C.  ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 17 June 2020 (17.06.2020) | 30 June 2020 (30.06.2020) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

## INTERNATIONAL SEARCH REPORT
Information on patent family members

International application No.

PCT/JP2020/013640

| Patent Documents referred in the Report | Publication Date | Patent Family | Publication Date |
|---|---|---|---|
| JP 2000-210264 A | 02 Aug. 2000 | JP 2006-239444 A<br>entire text, all drawings<br>US 6374128 B1<br>entire text, all drawings<br>EP 1002497 A2 | |
| JP 60-203235 A | 14 Oct. 1985 | (Family: none) | |
| JP 60-203236 A | 14 Oct. 1985 | (Family: none) | |
| WO 2019/035175 A1 | 21 Feb. 2019 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016027337 A

**Non-patent literature cited in the description**

- **MASAKI GOMA.** The Development of Small Laser Doppler Blood Flow Sensor. PIONEER R&D, 2012, vol. 21, 30-37